(19) Europäisches Patentamt
European Patent Office
Office européen des brevets

(11) **EP 2 873 387 A1**

(12) **EUROPEAN PATENT APPLICATION**

(43) Date of publication:
20.05.2015 Bulletin 2015/21

(51) Int Cl.:
**A61B 19/00** (2006.01)　　**A61B 17/3203** (2006.01)
**A61B 17/00** (2006.01)

(21) Application number: **14192996.8**

(22) Date of filing: **13.11.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.11.2013　JP 2013235681**

(71) Applicant: **Seiko Epson Corporation**
**Shinjuku-ku**
**Tokyo 163-0811 (JP)**

(72) Inventors:
• **Uchida, Kazuaki**
**Nagano, 392-8502 (JP)**
• **Kojima, Hideki**
**Nagano, 392-8502 (JP)**
• **Sekino, Hirokazu**
**Nagano, 392-8502 (JP)**
• **Seto, Takeshi**
**Nagano, 392-8502 (JP)**

(74) Representative: **Hoffmann Eitle**
**Patent- und Rechtsanwälte PartmbB**
**Arabellastraße 30**
**81925 München (DE)**

(54) **Robotic surgical apparatus and fluid ejecting apparatus for robotic surgical apparatus**

(57)　A robotic surgical apparatus, provided with a robot arm portion, an operation portion that receives an input by a user for operating the robot arm portion, and a tool control portion configured to operate the robot arm portion based on the input which the operation portion receives from a user, includes: a fluid chamber; a pulsating flow-provision portion configured to provide a fluid within the fluid chamber with a pulsating flow; a fluid ejection tube which includes a fluid ejection port for ejecting a fluid; a fluid supply portion which supplies a fluid to the fluid chamber; and a fluid ejection control portion which controls the pulsating flow-provision portion. The fluid ejection tube is mechanically fixed to the robot arm portion, and the fluid ejection control portion is configured such that the ejection amount of a fluid per unit time can be changed in accordance with the movement speed of the fluid ejection port.

FIG. 3

**Description**

<u>BACKGROUND</u>

1. Technical Field

The present invention relates to a robotic surgical apparatus capable of performing discission, resection, or the like on living tissue by ejecting a fluid, and to a fluid ejecting apparatus for the robotic surgical apparatus which is used as a portion of the robotic surgical apparatus and is capable of performing discission, resection, or the like on living tissue by ejecting a fluid.

2. Related Art

In the fluid ejecting apparatus used as a medical instrument, a technique of detecting operation acceleration of an operation portion, to which fluid is communicated, using an acceleration sensor, and of selecting a mode of fluid ejecting based on its acceleration is known (JP-A-2012-143374).

The related art performs discission, resection, or the like according to an intention of an operator by switching the ejection mode depending on a movement speed of the operation portion of the fluid ejecting apparatus. The inventors have found a method of further improving the technology and of favorably changing incising capability depending on the movement speed of a fluid ejection port.

In a minimally invasive medical technique, reduction in an amount of heterogeneous tissue which is damaged during diagnostic or surgical procedures is intended. Accordingly, recovery time of a patient, and unpleasant and harmful side effects are reduced. One of the effects of the minimally invasive surgery is, for example, reduction of postoperative recovery time during hospitalization. As the minimally invasive medical technique, a robotic surgical apparatus been developed. For example, in the robotic surgical apparatus, there is a case in which a surgeon does not move the surgical instruments by directly carrying those by hand, but instead, the movement of surgical instruments is operated by operating an operation portion associated with surgical instruments and a servomechanism.

The method of performing discission, resection, or the like on living tissue by ejecting a fluid is also a technique enabling minimally invasive treatment, and it is possible to provide a minimally invasive medical technique which is not in the related art by combining the fluid ejecting apparatus, which can favorably change the incising capability depending on the movement speed of the fluid ejection port, with the above-described robotic surgical apparatus.

<u>SUMMARY</u>

An advantage of some aspects of the invention is to solve at least one of the problems described above and the invention can be implemented as the following aspects.

(1) An aspect of the invention provides a robotic surgical apparatus.

The robotic surgical apparatus, which is provided with a robot arm portion, an operation portion that receives an input by a user for operating the robot arm portion, a tool control portion configured to operate the robot arm portion based on the input which the operation portion receives from a user, and a fluid ejecting portion configured to eject a fluid jet from a nozzle thereof, the fluid ejecting portion comprising: a pulsating flow-provision portion including a fluid chamber and configured to provide a fluid within the fluid chamber with a pulsating flow; a fluid ejection tube which includes a fluid ejection port for ejecting a fluid; a fluid supply portion which supplies a fluid to the fluid chamber; and a fluid ejection control portion which controls the pulsating flow-provision portion, in which the fluid ejection tube is mechanically fixed to the robot arm portion, and the fluid ejection control portion is configured such that the ejection amount of a fluid per unit time can be changed in accordance with the movement speed of the fluid ejection port. According to the aspect of the invention, the ejection amount of a fluid per unit time can be changed in accordance with the movement speed of the fluid ejection port, and therefore, it is possible to adjust resecting capability in accordance with the movement speed of the fluid ejection port.

(2) In the robotic surgical apparatus according to the aspect of the invention, the pulsating flow-provision portion may include a capacity variation portion that changes the capacity in the fluid chamber. The robotic surgical apparatus may further include a fluid supply portion which supplies a fluid to the fluid chamber; and a control portion which controls the capacity variation portion and the fluid supply portion. The control portion may change at least one of a voltage applied to the capacity variation portion and a flow rate of a fluid supplied to the fluid chamber in accordance with the movement speed of the fluid ejection port. According to this configuration, at least any one of the voltage associated with the depth of resection and the supplied flow rate (hereinafter, also referred to as "supply flow rate") changes in accordance with the movement speed of the fluid ejection port, and therefore, it is possible to adjust the resecting capability in accordance with the movement speed of the fluid ejection port.

(3) In the robotic surgical apparatus according to the aspect of the invention, the control portion may change the voltage and the flow rate. According to this configuration, both of the voltage and the flow rate of the fluid change in accordance with the movement speed of the fluid ejection port, and therefore, it is possible to adjust the resecting capability in accordance with the movement speed of the fluid ejection port and to implement control of the flow rate depending on the change of the voltage.

(4) In the robotic surgical apparatus according to the aspect of the invention, the control portion may set the voltage to a first voltage when the movement speed of the fluid ejection port is a first speed, and set the voltage to a second voltage higher than the first voltage when the movement speed is a second speed faster than the first speed. According to this configuration, if the movement speed of the fluid ejection port becomes fast, the voltage changes so as to improve the resecting capability, and therefore, it is possible to stabilize the depth of resection.

(5) In the robotic surgical apparatus according to the aspect of the invention, the control portion may set the flow rate of the fluid supplied to the fluid chamber to a first flow rate when the movement speed is the first speed, and set the flow rate of the fluid supplied to the fluid chamber to a second flow rate larger than the first flow rate when the movement speed is the second speed. According to this configuration, the supply flow rate increases if the movement speed becomes fast, and therefore, it is possible to stabilize the depth of resection. Furthermore, according to this configuration, if the voltage becomes high, the supply flow rate increases. If the voltage becomes high, a required flow rate increases. Thus, according to this configuration, there is a high possibility that an adequate supply flow rate can be secured when there is a change in the required flow rate.

(6) In the robotic surgical apparatus according to the aspect of the invention, a driving signal may be applied to the capacity variation portion. The control portion may change the voltage at the movement speed lower than or equal to a third speed which is faster than the second speed, set the frequency of the driving signal to a first frequency when the movement speed is the third speed, and set the frequency of the driving signal to a second frequency which is higher than the first frequency when the movement speed is a fourth speed which is faster than the third speed. According to this configuration, if the movement speed becomes fast, the drive frequency changes so as to improve the resecting capability, and therefore, it is possible to stabilize the depth of resection. Furthermore, it is possible to determine the drive frequency by excluding the influence caused by the change of the voltage if the voltage does not change while the drive frequency can change at the third speed or higher.

(7) In the robotic surgical apparatus according to the aspect of the invention, the control portion may perform control of the voltage, the flow rate, and a frequency of the driving signal in accordance with the movement speed. According to this configuration, it is possible to adjust the resecting capability in accordance with the movement speed of the fluid ejection port using the three parameters.

(8) In the robotic surgical apparatus according to the aspect of the invention, the robot surgical apparatus may further include a pulsating-flow provision portion including a pressurization portion that pressurizes the inside of the fluid chamber; and a control portion that changes a driving signal transmitted to the pressurization portion in accordance with the movement speed of the fluid ejection port. According to this configuration, it is possible to change the driving signal transmitted to the pressurization portion in accordance with the movement speed of the fluid ejection port.

(9) In the robotic surgical apparatus according to the aspect of the invention, the robotic surgical apparatus may further include The control portion may change time until the driving signal reaches a second predetermined voltage from a first predetermined voltage in accordance with the movement speed of the fluid ejection port. According to this configuration, a startup time (time until the driving signal reaches the second predetermined voltage from the first predetermined voltage) changes in accordance with the movement speed, and thus, the depth of resection is stabilized. This is because the startup time is a parameter associated with the depth of resection.

(10) In the robotic surgical apparatus according to the aspect of the invention, the control portion may set the startup time to a first time when the movement speed is a first speed, and set the startup time to a second time shorter than the first time when the movement speed is a second speed faster than the first speed. The previous aspect can be implemented in the manner of this configuration.

(11) In the robotic surgical apparatus according to the aspect of the invention, the control portion may set a maximum voltage of the driving signal to a first voltage when the movement speed is the second speed, and set the maximum voltage of the driving signal to a second voltage higher than the first voltage when the movement speed is a third speed faster than the second speed. According to this configuration, the maximum voltage of the driving signal changes in accordance with the movement speed, and thus, the depth of resection is stabilized. This is because the maximum voltage of the driving signal is a parameter associated with the depth of resection.

(12) In the robotic surgical apparatus according to the aspect of the invention, the control portion may set the flow rate of the fluid to a first flow rate when the movement speed is the second speed, and set the flow rate of the fluid to a second flow rate larger than the first flow rate when the movement speed is the third speed which is larger than the second speed. According to this configuration, the flow rate of the fluid is set to the first flow rate when the maximum voltage of the driving signal is the first voltage and is set to the second flow rate when the maximum voltage of the driving signal is the second voltage, and therefore, it is possible to change the flow rate in accordance with the maximum voltage.

(13) In the robotic surgical apparatus according to the aspect of the invention, when the movement speed is a fourth speed faster than the third speed, the control portion may set the startup time to a third time shorter than the second time and set a maximum voltage of the driving signal to a third voltage higher than the second voltage. According to this configuration, even if the movement speed becomes fast up to the fourth speed, the depth of resection is easily stabilized.

(14) In the robotic surgical apparatus according to the aspect of the invention, the control portion may set the flow rate of the fluid to a third flow rate when the movement speed is the fourth speed which is larger than the third speed. According to this configuration, when the movement speed is the fourth speed, it is possible to set the flow rate to be different from a case in which the movement speed is lower than or equal to the third speed.

(15) In the robotic surgical apparatus according to the aspect of the invention, when the movement speed is a first predetermined speed slower than the first speed and is a second predetermined speed slower than the first predetermined speed, the control portion may set the startup time, the maximum voltage of the driving signal, and the flow rate of the fluid to predetermined values respectively. According to this configuration, when the movement speed is lower than or equal to a reference value, it is possible to set the startup time, the maximum voltage, and the flow rate of the fluid to predetermined values.

(16) In the robotic surgical apparatus according to the aspect of the invention, the pulsating flow-provision portion may include an output portion that outputs a laser beam into the fluid chamber and a control portion that changes a driving signal transmitted to the output portion in accordance with the movement speed of the fluid ejection port. According to this configuration, the driving signal is changed in accordance with the movement speed. Therefore, it is possible to adjust the output of the laser beam in accordance with the movement speed and to stabilize the depth of resection.

(17) In the robotic surgical apparatus according to the aspect of the invention, the control portion may set the voltage of the driving signal to a first voltage when the movement speed is a first speed, and set the voltage of the driving signal to a second voltage higher than the first voltage when the movement speed is a second speed faster than the first speed. According to this configuration, it is possible to easily control the output of the laser beam. This is because it is comparatively easy to increase the energy of the laser beam per output.

(18) In the robotic surgical apparatus according to the aspect of the invention, the control portion may change the frequency of the driving signal in accordance with the movement speed. According to this configuration, it is possible to adjust the output of the laser beam through methods other than changing the maximum voltage.

(19) In the robotic surgical apparatus according to the aspect of the invention, the control portion may set the frequency of the driving signal to a first frequency when the maximum voltage is the first and the second voltages, and set the frequency of the driving signal to a second frequency higher than the first frequency when the maximum voltage is a third voltage higher than the second voltage. According to this configuration, when the maximum voltage is the first and the second voltages, the output is controlled by changing the maximum voltage without changing the frequency of the driving signal, and therefore, it is easy to determine the values such as the first and the second voltages.

(20) In the robotic surgical apparatus according to the aspect of the invention, when the movement speed is a third speed faster than the second speed, the control portion may set the maximum voltage to the third voltage and set the frequency of the driving signal to the second frequency, and when the movement speed is a fourth speed faster than the third speed, the maximum voltage is set to the third voltage and the frequency of the driving signal is set to a third frequency higher than the second frequency. According to this configuration, the output is controlled by changing the frequency without changing the maximum voltage of the driving signal when the movement speed is the third and fourth speeds, and therefore, it is easy to determine the values such as the second and the third frequencies.

(21) In the robotic surgical apparatus according to the aspect of the invention, the robotic surgical apparatus may include the fluid supply portion which supplies a fluid to the fluid chamber at a flow rate which is set by the control portion. The control portion may set the flow rate to a first flow rate when the movement speed is the first speed, and set the flow rate to a second flow rate larger than the first flow rate when the movement speed is the second speed. According to this configuration, it is possible to adequately set the flow rate to be supplied.

(22) In the robotic surgical apparatus according to the aspect of the invention, the control portion may change the maximum voltage and the frequency of the driving signal in accordance with the movement speed. According to this configuration, it is possible to change the output of the laser beam depending on the maximum voltage and the frequency of the driving signal.

(23) Another aspect of the invention provides a fluid ejecting apparatus for a robotic surgical apparatus.

The fluid ejecting apparatus for robotic surgery is a fluid ejecting apparatus for a robotic surgical apparatus comprising a pulsating flow-provision portion including a fluid chamber and configured to provide a fluid within the fluid chamber with a pulsating flow; a fluid ejection port for ejecting a fluid; a fluid ejection tube which includes a fluid ejection port for ejecting a fluid; and a fluid ejection control portion which controls the pulsating flow-provision portion. The fluid ejection tube can be mechanically fixed to a robot arm portion of the robotic surgical apparatus, and the fluid ejection control portion is configured such that the ejection amount of a fluid per unit time can be changed in accordance with the movement speed of the fluid ejection port. According to the aspect of the invention, it is possible to change the ejection amount of the fluid per unit time in accordance with the movement speed of the fluid ejection port, and thus, it is possible to adjust the resecting capability in accordance with the movement speed of the fluid ejection port.

BRIEF DESCRIPTION OF THE DRAWINGS

The invention will be described with reference to the accompanying drawings, wherein like numbers reference like elements.

Fig. 1 is a schematic plan view of a part of surgical theater showing a system using a robot.
Fig. 2 is a perspective view of a patient-side robot cart or stand.
Fig. 3 is a configuration view of a fluid ejecting apparatus.
Fig. 4 is an inner structural view of a fluid ejection mechanism.
Fig. 5 is a graph showing a drive waveform.
Fig. 6 is a flowchart showing ejection treatment (Embodiment 1).
Figs. 7A and 7B are graphs showing a relationship between parameters and a movement speed (Embodiment 1).
Fig. 8 is a graph showing a state in which drive waveforms change.
Fig. 9 is a graph showing a relationship between a supply flow rate and a demand flow rate.
Fig. 10 is a graph showing a relationship between a demand flow rate and a peak voltage.
Fig. 11 is a graph showing a relationship between a demand flow rate and a drive frequency.
Fig. 12 is a graph showing a relationship between an ejection pressure and a peak voltage.
Fig. 13 is a graph showing a relationship between the depth of resection and a peak voltage.
Fig. 14 is a flowchart showing ejection treatment (Embodiment 2).
Figs. 15A and 15B are graphs showing a relationship between parameters and a movement speed (Embodiment 3).
Fig. 16 is a configuration view of a fluid ejecting apparatus.
Fig. 17 is an inner structural view of a fluid ejection mechanism.
Fig. 18 is a flowchart showing ejection treatment (Embodiment 4).
Fig. 19 is a graph showing a waveform of one period of drive waveforms (Embodiment 4).
Fig. 20 is a graph showing a relationship between a startup time Tr and a speed S (Embodiment 4).
Fig. 21 is a graph showing a relationship between a peak voltage Vp and a speed S (Embodiment 4).
Fig. 22 is a graph showing a technique of determining a flow rate (Embodiment 4).
Fig. 23 is a table showing a test result in which a relationship between a startup time, a maximum pressure of an ejected fluid, and change of the depth of resection is examined.
Fig. 24 is a configuration view of a fluid ejecting apparatus.
Fig. 25 is a cross-sectional view showing the inside of a fluid ejection mechanism.
Fig. 26 is a graph showing a waveform of a driving signal.
Fig. 27 is a flowchart showing ejection treatment.
Figs. 28A and 28B are graphs showing relationships between a drive voltage and a movement speed and between a drive frequency and a movement speed.
Fig. 29 is a graph showing a relationship between a drive frequency and a drive voltage (Embodiment 5).
Fig. 30 is a flowchart showing ejection treatment (Embodiment 6).

DESCRIPTION OF EXEMPLARY EMBODIMENTS

Embodiment 1

Embodiment 1 will be described.

A robotic surgical apparatus will be described. In robotic surgery, a surgeon does not move instruments by directly carrying those by hand, for example, the movement of the surgical instruments is operated using a particular form such as a servomechanism. A surgeon is provided with an image of a surgical site in a surgical workstation and implements a surgical procedure on a patient by operating a master control device while watching two- or three-dimensional images of the surgical site on the display. Manipulation of the master control device controls the movement of instruments operated by a servomechanism.
A servomechanism used for the robotic surgery frequently can receive an input from two master controllers (for each hand of a surgeon) and can include two or more robot arms to which surgical instruments are respectively attached. Communication between operational signals among master controllers, associated robot arms, and instrument assemblies are typically achieved through a control system. The control system typically includes at least one processor.

The control system sends an input command from a master controller to associated robot arms and instrument assemblies, and, for example, in a case of performing feedback or other cases, the control system performs relay by returning the input command to the associated master controller from the instruments and arm assemblies.

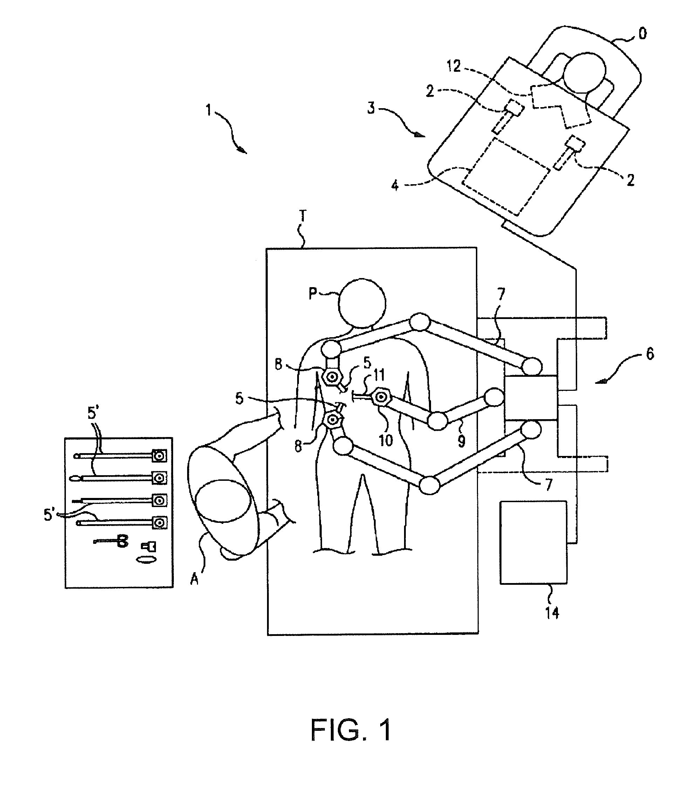

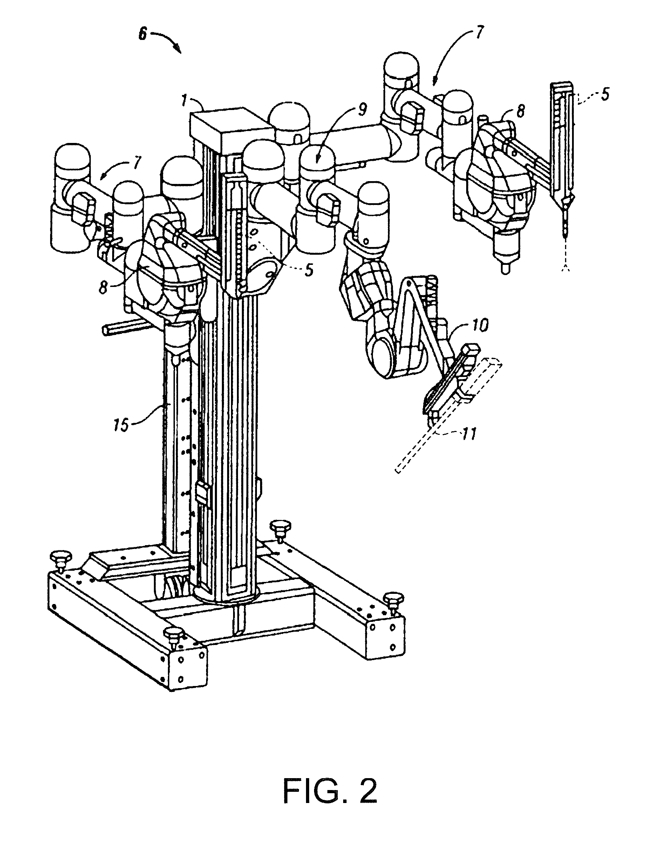

Figs. 1 and 2 show a robotic surgical system 1 for implementing minimally invasive robotic surgery. An operator O (generally, a surgeon) performs a minimally invasive surgical procedure on a patient P lying on an operating table T. The operator O operates one or more input devices or masters 2 on a console 3 for a doctor. A computer processor 4 of the console 3 instructs a movement of an endoscopic instrument or a tool 5 in response to an input of a surgeon, and performs servomechanism movement of instruments through a patient-side robotic system 6 (in this case, a system mounted on a cart).

Typically, the patient-side system or cart 6 includes at least three robot manipulator arms. Two arms or connection portions 7 (which are mounted on a side of the cart 6 in this case) support and position servo-manipulators 8, and drive a surgical tool 5. Moreover, an arm or connection portion 9 (which are mounted in the center of the cart 6 in this case) supports and positions a servo-manipulator 10, and controls movement of an endoscope/camera probe 11 that captures an image of an internal surgical site (preferably, a stereoscopic image).

The image of the internal surgical site is shown to a surgeon or operator O, through a stereoscopic display viewer 12 in the console 3 for a doctor, and is simultaneously shown to an assistant A through a display 14 for an assistant. The assistant A supports prepositioning of manipulators 8 and 10 for the patient P using the setup connection arms 7 and 9 by exchanging the tool 5 in one or more surgical manipulators 8 (and/or 10) for alternative tools or instruments 5' and by operating associated manual medical instruments and apparatuses.

Roughly speaking, the arms or connection portions 7 and 9 typically include positioning connection portions or setup arm portions of the patient-side system 6 in a fixed form while the system is operated, and the manipulators 8 and 10 include a driving portion that actively articulates under the instruction of the console 3 from a surgeon. In the present specification, the portion which is actively driven is generally called a "manipulator" and the portion that can fix the positioning connections of the patient-side system is called a "setup arm". In addition, it is denoted that such a setup arm is supplied with power as necessary and can have a joint which is controlled by a computer.

For convenience of terminology, in the present specification, the manipulator such as 8 which acts on the system that affects the surgical tools is referred to as a patient-side manipulator (PSM), and the manipulator such as 10 which controls an image-capturing or data-obtaining device such as the endoscope 11 is generally referred to as an endoscope-camera manipulator (ECM). It is denoted that such a robotic manipulator manipulates and controls instruments, tools, and devices in a wide and useful range for surgery as necessary.

Fig. 2 shows a perspective view of a robotic surgical patient-side system 6 mounted on a cart of Fig. 1, and includes two PSMs 8 and an ECM 10. The cart system 6 includes a column 15 and is mounted with three positioning connection portions or setup arms, each of which includes two PSM setup arms 7 each of which supports one of the PSMs 8 and a setup arm 9 that supports the ECM 10. Each of the PSM setup arms 7 has six degrees of freedom and is mounted on each side surface of the ECM setup arm 9 which is mounted on the center. The ECM setup arm 9 has degrees of freedom less than six, and the ECM 10 may not include a whole tool operation drive system which is typically included in the PSM 8 and is provided for surgical instruments having joints. Each PSM 8 is detachably mounted with the surgical tool 5 (which is shown by a dotted line) and the ECM 10 is detachably mounted with the endoscope probe 11 (which is shown by a dotted line).

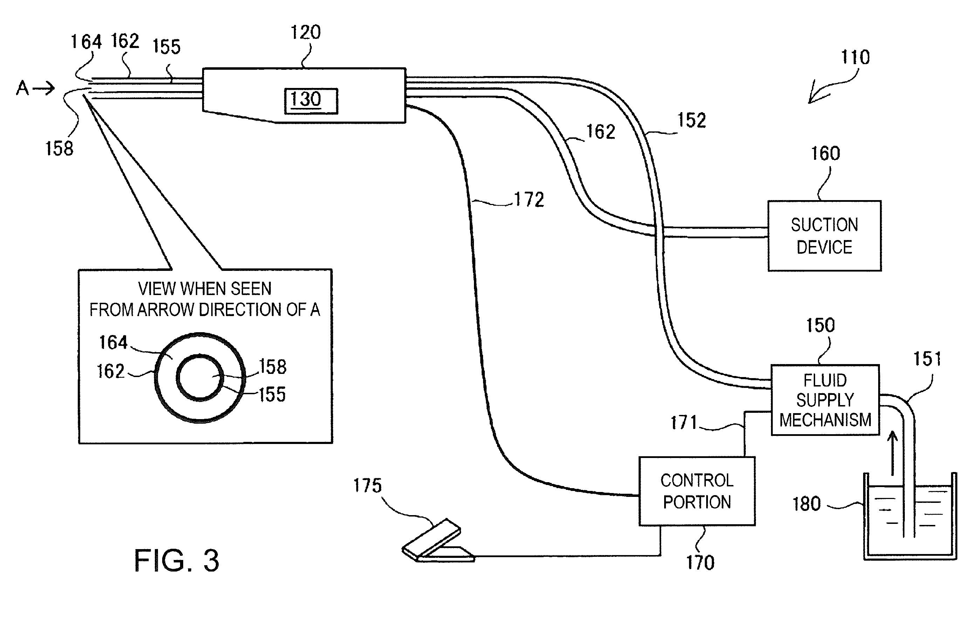

Fig. 3 shows a configuration of the fluid ejecting apparatus 110 including a fluid ejection mechanism 120 as the surgical tool 5 included in the patient-side system of the robotic surgical apparatus. The fluid ejecting apparatus 110 is a medical instrument used in medical institutions and has a function of making an incision or resecting a lesion portion by ejecting a fluid to the lesion portion. In the present specification, the robotic surgical apparatus indicates a robotic surgical apparatus including the following fluid ejection mechanism or a robotic surgical apparatus including the fluid ejecting apparatus including the following fluid ejection mechanism. In addition, the fluid ejecting apparatus for the robotic surgical apparatus indicates the following fluid ejecting apparatus which can be used by being embedded in the robotic surgical apparatus.

The fluid ejecting apparatus 110 includes a fluid ejection mechanism 120, a fluid supply mechanism 150, a suction device 160, a control portion 170, and a fluid container 180. The fluid supply mechanism 150 and the fluid container 180 are connected to each other through a connection tube 151. The fluid supply mechanism 150 and the fluid ejection mechanism 120 are connected to each other through a fluid supply passage 152. The connection tube 151 and the fluid supply passage 152 are formed of resin. The connection tube 151 and the fluid supply passage 152 may be formed of materials other than resin (for example, metal).

The fluid container 180 stores physiological saline. Pure water or fluid medicine may be used instead of the physiological saline. The fluid supply mechanism 150 supplies the fluid ejection mechanism 120 with a fluid, which is sucked from the fluid container 180 via the connection tube 151, via the fluid supply passage 152 through driving of a built-in pump.

The fluid ejection mechanism 120 is an instrument which a user of the fluid ejecting apparatus 110 operates by hand. The user performs discission or resection on a lesion portion by bringing a fluid, which is intermittently ejected from an ejection port 158, into contact with the lesion portion.

The control portion 170 transmits a driving signal to a pulsation generation portion 130 which is built into the fluid ejection mechanism 120 through a signal cable 172. The control portion 170 controls the flow rate of a fluid which is supplied to the pulsation generation portion 130 by controlling the fluid supply mechanism 150 through a control cable 171. A foot switch 175 is connected to the control portion 170. When a user turns on the foot switch 175, the control portion 170 performs supplying of a fluid to the pulsation generation portion 130 by controlling the fluid supply mechanism 150 and generates pulsation on the pressure of the fluid supplied to the pulsation generation portion 130 by transmitting the driving signal to the pulsation generation portion 130. The mechanism of generating the pulsation and the control of ejecting a fluid from the fluid ejection mechanism 120 will be described in detail later.

The suction device 160 sucks a fluid or resected substance in the periphery of the ejection port 158. The suction device 160 and the fluid ejection mechanism 120 are connected to each other through a suction passage 162. The suction device 160 sucks the inside of the suction passage 162 at all times while a switch for operating the suction device 160 is turned on. The suction passage 162 is opened in the vicinity of a tip end of an ejection tube 155 by passing through the fluid ejection mechanism 120.

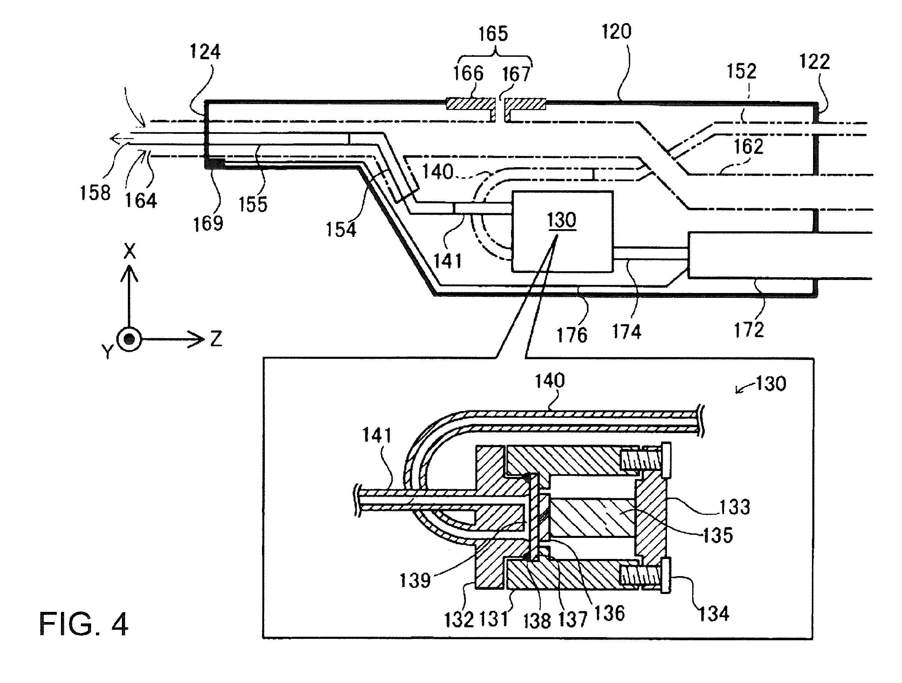

The suction passage 162 covers the ejection tube 155 extending from a tip end of the fluid ejection mechanism 120. For this reason, as shown in a view when seen from the arrow direction of A of Fig. 3, the wall of the ejection tube 155 and the wall of the suction passage 162 form approximately concentric cylindrical shapes. A passage, through which a sucked substance which is sucked from a suction port 164 as a tip end of the suction passage 162 flows, is formed between the outer wall of the ejection tube 155 and the inner wall of the suction passage 162. The sucked substance is sucked into the suction device 160 through the suction passage 162. The suctioning is adjusted by a suction force adjustment mechanism 165 to be described later using Fig. 4.

Fig. 4 shows an internal structure of the fluid ejection mechanism 120. The fluid ejection mechanism 120 is equipped with the pulsation generation portion 130, an inlet passage 140, an exit passage 141, a connection tube 154, and an acceleration sensor 169 therein, and includes the suction force adjustment mechanism 165.

The pulsation generation portion 130 generates pulsation on the pressure of a fluid supplied from the fluid supply mechanism 150 to the fluid ejection mechanism 120 through the fluid supply passage 152. The fluid in which the pulsation of the pressure is generated is supplied to the ejection tube 155. The fluid supplied to the ejection tube 155 is intermittently ejected from the ejection port 158. The ejection tube 155 is formed of stainless steel. The ejection tube 155 may be formed of other materials, for example, other metals such as brass or reinforced plastics having rigidity at a predetermined level or higher.

As shown in the enlarged view at a lower part of Fig. 4, the pulsation generation portion 130 includes a first case 131, a second case 132, a third case 133, a bolt 134, a piezoelectric element 135, a reinforcing plate 136, a diaphragm 137, a packing 138, the inlet passage 140, and the exit passage 141. The first case 131 is a cylindrical member. The second case 132 is connected to one end portion of the first case 131, and the third case 133 is fixed to the other end portion thereof using the bolt 134. Therefore, the entire first case is sealed. The piezoelectric element 135 is disposed in a space which is formed inside the first case 131.

The piezoelectric element 135 is a laminated piezoelectric element. An end of the piezoelectric element 135 is fixed to the diaphragm 137 through the reinforcing plate 136. The other end of the piezoelectric element 135 is fixed to the third case 133. The diaphragm 137 is made of a metallic thin film. The periphery of the diaphragm 137 is fixed to the first case 131 and is interposed between the first case 131 and the second case 132. A fluid chamber 139 is formed between the diaphragm 137 and the second case 132.

A driving signal is input from the control portion 170 to the piezoelectric element 135 through the signal cable 172. The signal cable 172 is inserted from a rear end portion 122 of the fluid ejection mechanism 120. The signal cable 172 accommodates two electrode wires 174 and a signal line 176 for the acceleration sensor. The electrode wires 174 are connected to the piezoelectric element 135 in the pulsation generation portion 130. The piezoelectric element 135 expands and contracts based on the driving signal which is transmitted from the control portion 170. The capacity of the fluid chamber 139 fluctuates depending on the expansion and contraction of the piezoelectric element 135.

The inlet passage 140 into which a fluid flows is connected to the second case 132. The inlet passage 140 is bent in a U shape and extends toward the rear end portion 122 of the fluid ejection mechanism 120. The fluid supply passage 152 is connected to the inlet passage 140. The fluid which is supplied from the fluid supply mechanism 150 is supplied to the fluid chamber 139 through the fluid supply passage 152.

When the piezoelectric element 135 expands and contracts at a predetermined frequency, the diaphragm 137 vibrates. When the diaphragm 137 vibrates, the capacity of the fluid chamber 139 fluctuates and the pressure of the fluid in the fluid chamber is pulsated. The pressurized fluid flows out from the exit passage 141 which is connected to the fluid chamber 139.

The ejection tube 155 is connected to the exit passage 141 through a metallic connection tube 154. The fluid flowing out to the exit passage 141 is ejected from the ejection port 158 through the connection tube 154 and the ejection tube 155.

The suction force adjustment mechanism 165 adjusts a force by which the suction passage 162 sucks a fluid or the like from the suction port 164. The suction force adjustment mechanism 165 includes an operation portion 166 and a hole 167. The hole 167 is a through hole that connects between the suction passage 162 and the operation portion 166. When a user opens and closes the hole 167 using a finger of a hand holding the fluid ejection mechanism 120, the amount of air flowing into the suction passage 162 through the hole 167 is adjusted depending on the port and closing status. Accordingly, the suction force of the suction port 164 is adjusted. The adjustment of the suction force may be implemented through the control by the suction device 160.

The fluid ejection mechanism 120 includes the acceleration sensor 169. The acceleration sensor 169 is a piezo-resistance type triaxial acceleration sensor. The three axes are axes of X, Y, and Z shown in Fig. 4. The X-axis is parallel to the penetration direction of the hole 167 and the upward direction is a positive direction. The Z-axis is parallel to the long axis direction of the ejection tube 155 and a direction to which a fluid is ejected is set to be a negative direction. The Y-axis is defined by a right-hand system based on the X-axis and the Z-axis.

As shown in Fig. 4, the acceleration sensor 169 is disposed in the vicinity of a tip end portion 124 of the fluid ejection mechanism 120. The measurement result is input into the control portion 170 through the signal line 176 for the acceleration sensor.

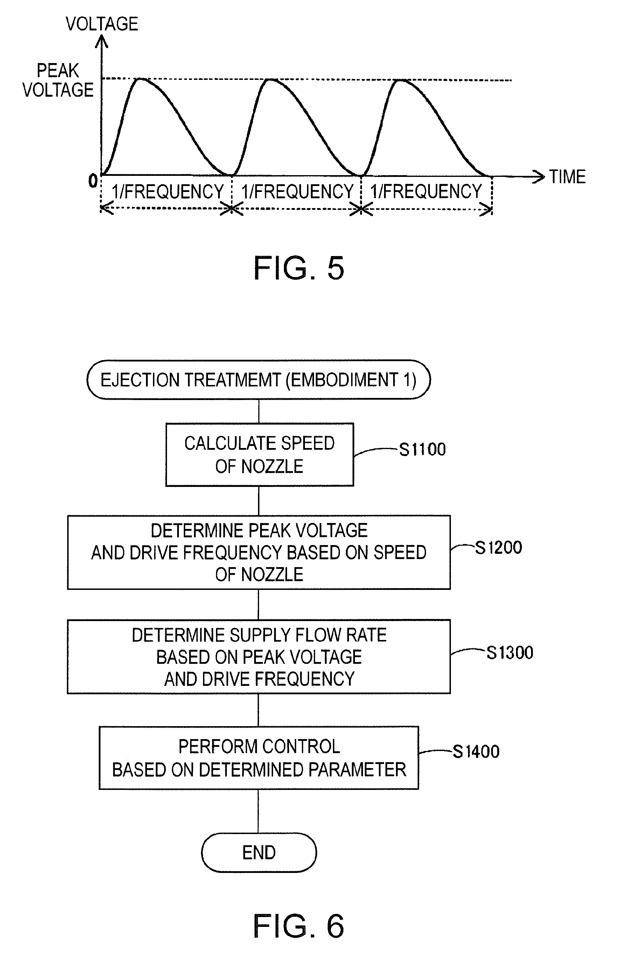

Fig. 5 is a graph showing a waveform of a driving signal (hereinafter, referred to as a "drive waveform") which is input into the piezoelectric element 135. The longitudinal axis indicates voltage and the horizontal axis indicates time. The drive waveforms are described as a combination of sine curves. The peak voltage and a frequency of the drive waveform are changed by ejection treatment (which will be described along with Fig. 6).

When the voltage value of the driving signal increases, the piezoelectric element 135 is deformed such that the fluid chamber 139 contracts in volume. The contraction repeatedly occurs as the driving signal is repeatedly input. As a result, the fluid is intermittently ejected.

Fig. 6 is a flowchart showing ejection treatment. The ejection treatment is repeatedly performed by the control portion 170 while the foot switch 175 is stepped on. First, a speed S of the ejection port 158 is calculated (step S1100). The speed S referred herein is an absolute value of the speed in an XY plane, that is, an absolute value of the speed in which the speed in the Z-axis direction is neglected. The speed S is calculated based on the triaxial acceleration measured by the acceleration sensor 169.

The speed S is calculated as a parameter that affects the depth of resection of a lesion portion. This is because the resecting capability that acts on each local area of a lesion portion per unit time is affected by a movement speed between the ejection port 158 and the lesion portion. In the present embodiment, the speed S is treated as the movement speed between the lesion portion and the ejection port 158 based on the assumption that the lesion portion is stationary. The speed S may be treated as a relative speed between the ejection port 158 and the lesion portion in consideration of a case in which the lesion portion is moved due to respiration or the like.

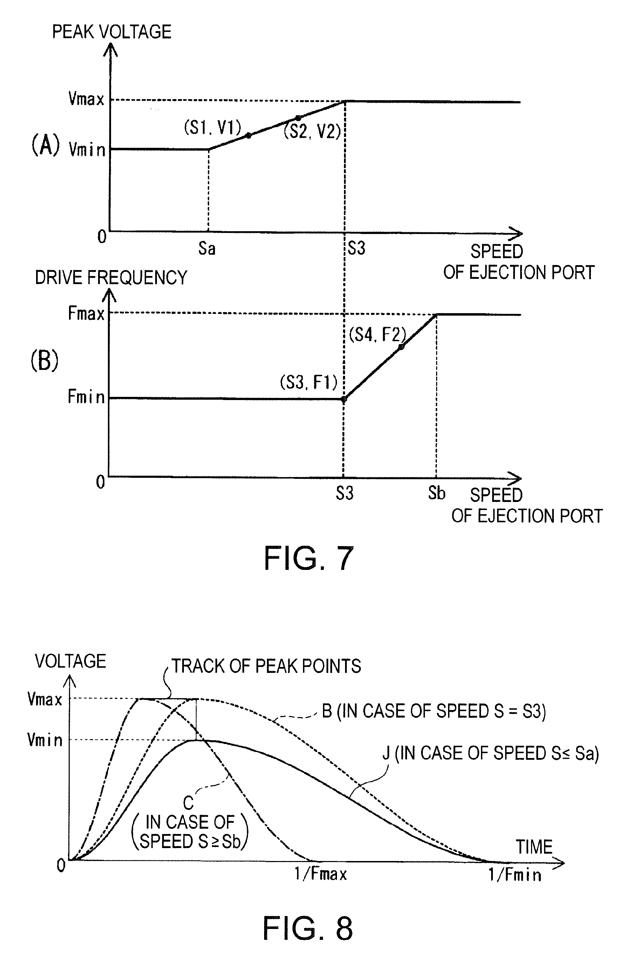

Subsequently, a peak voltage and a drive frequency are determined based on the calculated speed S (step S1200). Figs. 7A and 7B are graphs showing relationships between the peak voltage and the speed S and between the drive frequency and the speed S. Fig. 7A shows the peak voltage and Fig. 7B shows the drive frequency in the longitudinal axes. The horizontal axes thereof are commonly the speed S, and scales in Figs. 7A and 7B are coincident.

As shown in Figs. 7A and 7B, in each of speed ranges which are Sa ≤ speed S ≤ S3 and S3 ≤ speed S ≤ Sb, the parameters by which the values change are different from each other. That is, Sa, S3, and Sb are speeds determined in advance as a threshold value for switching the parameters to be changed.

In a case of speed S ≤ Sa, the peak voltage is fixed to Vmin which is a minimum value and the drive frequency is fixed to Fmin which is a minimum value. In a case where the parameters are set in this manner, the resecting capability becomes lowest.

In a case of Sa ≤ speed S ≤ S3, the peak voltage linearly increases with respect to the increase of the speed S while the drive frequency is fixed to Fmin. In a case of speed S = S3, the peak voltage is set to Vmax which is a maximum value. Vmin is set such that the resecting capability is prevented from being excessively decreased. Vmax is set such that the load of the piezoelectric element 135 is prevented from being excessively increased.

In a case of S3 ≤ speed S ≤ Sb, the drive frequency linearly increases with respect to the increase of the speed S while the peak voltage is fixed to Vmax. In a case of speed S = Sb, the drive frequency is set to Fmax which is a maximum value. Fmin is set such that the resecting capability is prevented from being excessively decreased and intermittent ejection is implemented. Fmax is set such that the load of the piezoelectric element 135 is prevented from being excessively increased. A drive waveform changes when the peak voltage and the drive frequency change in this manner.

Fig. 8 is a graph showing a state in which drive waveforms change. The longitudinal axis indicates voltage and the horizontal axis indicates time. Fig. 8 exemplifies three drive waveforms. A curve J indicates a drive waveform in a case in which the peak voltage is set to Vmin and the drive frequency is set to Fmin. That is, the curve J indicates a drive waveform in the above-described case of speed S ≤ Sa. A curve B indicates a drive waveform in a case in which the peak voltage is set to Vmax and the drive frequency is set to Fmin. That is, the curve B indicates a drive waveform in the above-described case of speed S = S3. A curve C indicates a drive waveform in a case in which the peak voltage is set to Vmax and the drive frequency is set to Fmax. That is, the curve C indicates a drive waveform in the above-described case of speed S ≥ Sb.

When the peak voltage increases, the expansion and contraction amount of the piezoelectric element 135 increases, and therefore, the variation ratio of capacity variation of the fluid chamber 139 increases. The variation ratio is a value obtained by dividing a maximum capacity by a minimum capacity in the capacity variation. When the ratio of the capacity variation increases, pressure variation in the fluid chamber 139 increases. When the pressure variation in the fluid chamber 139 increases, the fluid is vigorously ejected. Furthermore, when the peak voltage increases, the amount of fluid to be ejected increases. Accordingly, the resecting capability increases when the peak voltage increases. As a result, even if the resecting capability that acts on a lesion portion per unit area is decreased by a speed S being fast, the decrease is offset, and thus, the depth of resection is stabilized. The offsetting referred to herein is not limited to the depth of resection not being changed at all even if the speed S changes, and includes at least a partial decrease of an influence due to the change of the speed S.

When the drive frequency increases, the number of times of ejecting a fluid per unit time increases. Furthermore, in a case of the present embodiment, as shown in Fig. 8, when the drive frequency increases, a startup time is shortened. The startup time is time until a voltage value of a driving signal reaches the peak from zero. When the startup time is shortened, the contraction of the fluid chamber 139 is performed over a short period of time. As a result, the fluid is vigorously ejected. Accordingly, when the drive frequency increases, the resecting capability increases, and therefore, the depth of resection is stabilized even if the speed S is fast.

As described above, a supply flow rate is determined after determining the peak voltage and the drive frequency (step S1300) and control is performed based on the determined peak voltage, drive frequency, and a supply flow rate (step S1400). The supply flow rate is a volume flow rate of a fluid supplied by the fluid supply mechanism 150.

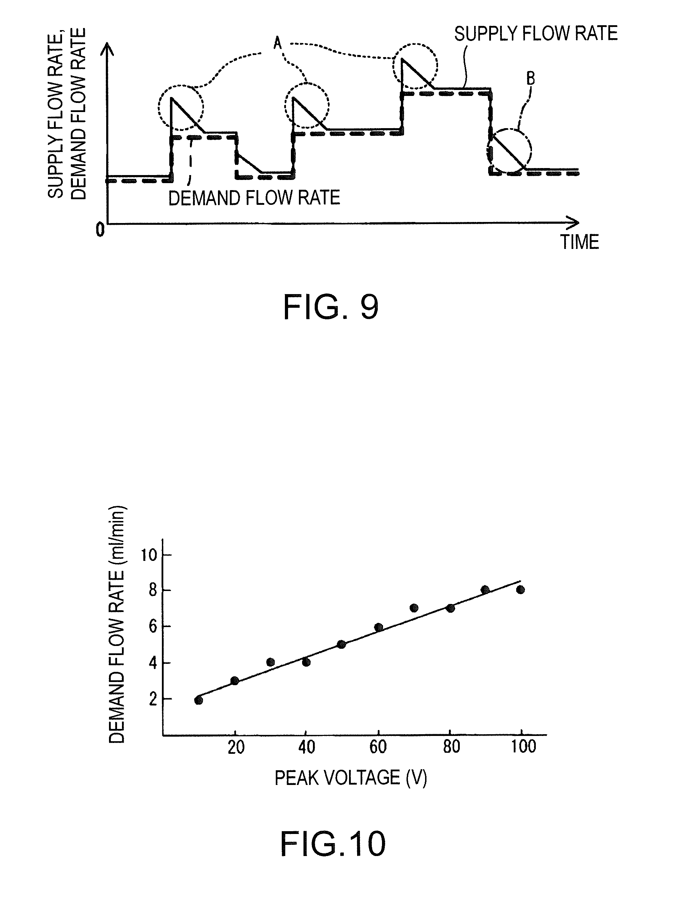

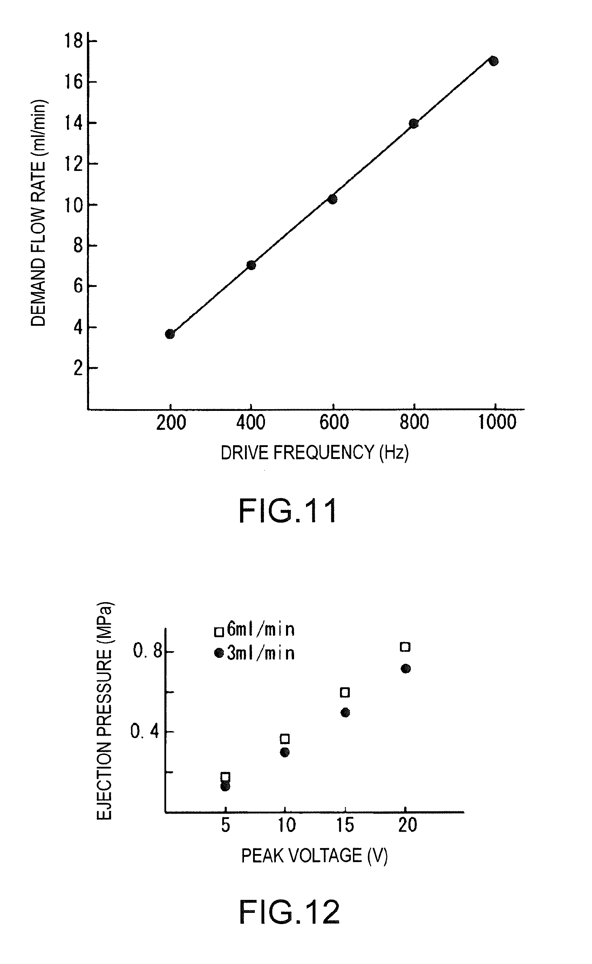

Fig. 9 is a graph schematically showing a method of determining the supply flow rate. The longitudinal axis indicates the supply flow rate and the demand flow rate and the horizontal axis indicates time. The demand flow rate is a flow rate required for filling the fluid chamber 139 with a fluid. A calculation method thereof will be described later along with Figs. 10 and 11.

The supply flow rate is basically set to a value slightly exceeding the demand flow rate. When the supply flow rate is lower than the demand flow rate, there is a possibility that ejection is not performed even if the fluid chamber 139 contracts in volume. When the ejection is not ordinarily performed in this way, in some cases, the resecting capability decreases. On the other hand, when the supply flow rate greatly exceeds the demand flow rate, there is a case where the fluid is ejected even at a timing at which the ejection should be suspended in order to implement intermittent ejection, and therefore, it may be difficult to ordinarily perform the intermittent ejection. Furthermore, when the supply flow rate greatly exceeds the demand flow rate, in some cases, the lesion portion is filled with the fluid, thereby interrupting the surgery. Accordingly, it is preferable that the supply flow rate be a value slightly exceeding the demand flow rate as described above.

In the present embodiment, when the demand flow rate is changed, the supply flow rate is made to be temporarily large. When the demand flow rate becomes 2 x Fd in a state in which the demand flow rate is Fd and the supply flow rate is Fs (>Fd), the supply flow rate is temporarily set to 3 x Fs, and then, is gradually converged to 2 x Fs. The portions represented by A in the curves indicating the supply flow rate of Fig. 9 schematically show the control of the flow rate.

Alternately, when the demand flow rate becomes 0.5 x Fd in a state in which the demand flow rate is Fd and the supply flow rate is Fs, the supply flow rate is temporarily set to 0.75 x Fs, and then, is gradually converged to 0.5 x Fs. The portion represented by B in the curves indicating the supply flow rate of Fig. 9 schematically shows the control of the flow rate.

In this manner, abnormal ejection of a fluid due to an insufficient supply flow rate caused by delay of control or undershooting is avoided by making the supply flow rate temporarily greater than the target value when the demand flow rate is changed.

Fig. 10 is a graph showing an experimental result with respect to a relationship between the demand flow rate and the peak voltage. Each point on the graph shows the experimental result and the straight line shows an approximate straight line of each point. As described in Fig. 10, when the peak voltage is doubled, the demand flow rate becomes about 1.5 times higher.

Fig. 11 is a graph showing an experimental result with respect to a relationship between the demand flow rate and the drive frequency. Each point on the graph shows the experimental result and the straight line shows an approximate straight line of each point. As described in Fig. 11, when the drive frequency is doubled, the demand flow rate is approximately doubled.

The determination of the supply flow rate in step S1300 shown in Fig. 6 is implemented by calculating the demand flow rate based on the relationships shown in Figs. 10 and 11 and by calculating the supply flow rate based on the calculated demand flow rate.

The supply flow rate also affects the resecting capability while the supply flow rate is determined based on the relationship with the demand flow rate as described above. Fig. 12 is a graph showing a relationship between an ejection pressure and the peak voltage when the supply flow rate is set in two ways. The drive frequency is set to an identical value in any case. Even in both cases in which the supply flow rate is 3 ml/min and 6 ml/min, the ejection pressure increases along with the increase of the peak voltage. This shows that the resecting capability is improved along with the increase of the above-described peak voltage.

As shown in Fig. 12, in each peak voltage, the ejection pressure in the case of 6 ml/min is higher than that in the case of 3 ml/min.

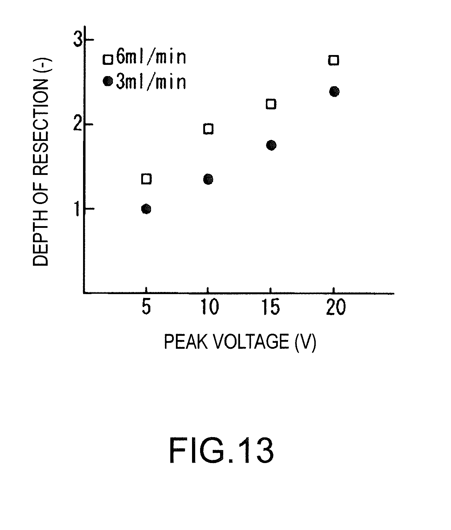

Fig. 13 is a graph showing a relationship between the depth of resection and the peak voltage when the supply flow rate is set in two ways. The depth of resection is represented by a value obtained by non-dimensionalization in which a value of a case where the peak voltage is 5 V and the supply flow rate is 3 ml/min is taken as 1. The drive frequency is set to an identical value in any case.

Similarly to the case of the ejection pressure (Fig. 12), even in any case where the supply flow rate is 3 ml/min or 6 ml/min, the depth of resection becomes deeper along with the increase of the peak voltage. In each peak voltage, the depth of resection in the case of 6 ml/min is deeper than that in the case of 3 ml/min.

Both graphs shown in Figs. 12 and 13 shows that the increase of the supply flow rate contributes to the improvement in the resecting capability. The above-described relationship between the speed S and the peak voltage and the relationship between the speed S and the drive frequency are determined by considering that the resecting capability is changed depending on the supply flow rate which is determined based on the relationship with the demand flow rate.

As described above, it is possible to stabilize the depth of resection by changing the peak voltage such that the resecting capability is improved along with the increase of the speed S. Furthermore, it is possible to stabilize the depth of resection by changing the drive frequency and the peak voltage when the peak voltage reaches a maximum value.

According to the present embodiment, it is easy to determine the values of the peak voltage and the drive frequency in each speed range since the ranges of the speed S that change the drive frequency and the peak voltage are separated from each other. Since the ranges of the speed S that change the peak voltage and the drive frequency are separated from each other, when the drive waveforms change, the peak points of the drive waveforms draw a track having a shape where a Γ-shape is rotated 90 degrees in a clockwise direction as shown in Fig. 8.

S1 to S4 shown in Figs. 7A and 7B are examples of first to fourth speeds in the appended claims, V1 and V2 shown in Fig. 7A are examples of first and second voltages in the appended claims, and F1 and F2 shown in Fig. 7B are examples of first and second frequencies in the appended claims. The piezoelectric element 135 and diaphragm 137 in the embodiment are examples of capacity variation portions in appended claims.

Embodiment 2

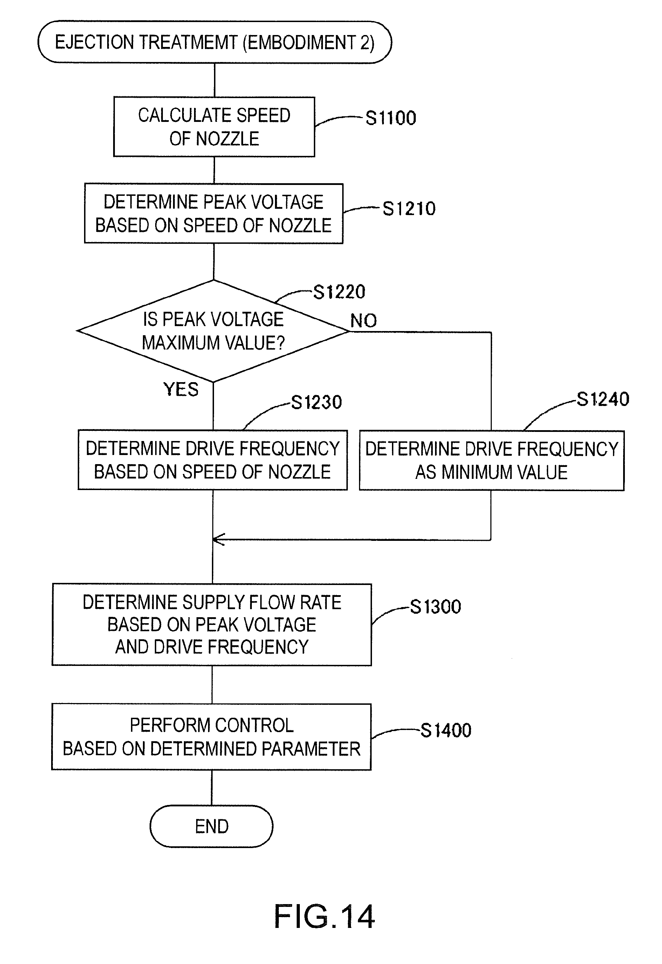

Embodiment 2 will be described. In Embodiment 2, ejection treatment shown in Fig. 14 is performed instead of the ejection treatment shown in Fig. 6. The description of the hardware configuration the same as that in Embodiment 1 will be omitted. The description of steps S1100, S1300, and S1400 in the ejection treatment of Embodiment 2 which are the same as those in Embodiment 1 will be omitted. In Embodiment 2, steps S1210 to S1240 are performed instead of step S1200 of Embodiment 1.

After calculating the speed S (step S1100), the peak voltage is determined based on the calculated speed S (step S1210). The method of determining the peak voltage is the same as that in Embodiment 1. That is, in a case of speed S ≤ S3', the peak voltage is fixed to Vmin, in a case of S3' < speed S ≤ S3, the peak voltage linearly increases, and in a case of S3 ≤ speed S, peak voltage is fixed to Vmax.

Next, it is determined whether the peak voltage is set to a maximum value (Vmax) (step S1220). When the peak voltage is set to a value lower than the maximum value (step S1220 : No), the drive frequency is set to a minimum value (Fmin) (step S1240). The setting of the peak voltage to the value lower than the maximum value means that there is a room for improvement in the resecting capability through the change of the peak voltage. Accordingly, it is unnecessary to improve the resecting capability by changing the value of the drive frequency, and therefore, the drive frequency is set to a minimum value.

On the other hand, when the peak voltage is set to a maximum value (step S1220: Yes), the drive frequency is determined based on the speed S (step S1230). The setting of the peak voltage to the maximum value means that there is no room for improvement in the resecting capability through the change of the peak voltage. Therefore, step S1230 is performed in order to improve the resecting capability by changing the value of the drive frequency. In Embodiment 2, it is also possible to obtain the control result the same as that in Embodiment 1.

Embodiment 3

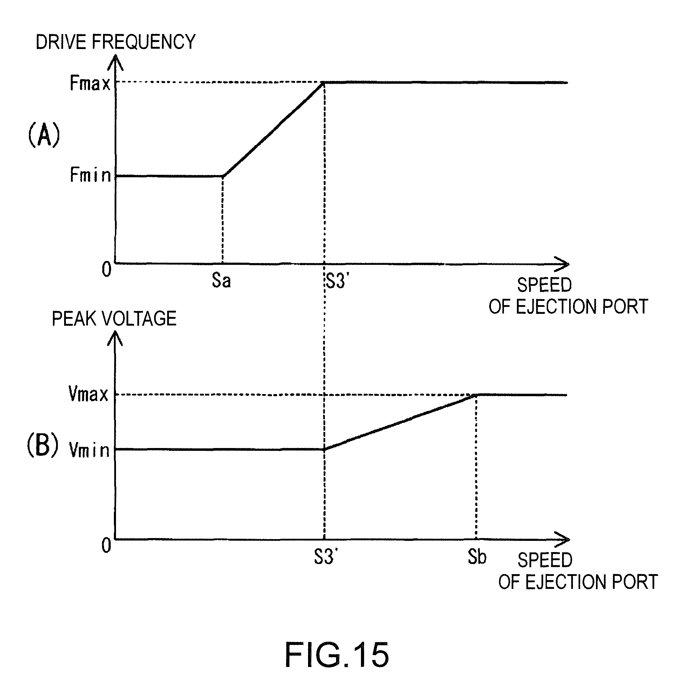

Embodiment 3 will be described. In Embodiment 3, step S1200 of ejection treatment is performed based on the relationship shown in Figs. 15A and 15B instead of the relationships between the peak voltage and the speed S and between the drive frequency and the speed S in Embodiment 1 shown in Figs. 7A and 7B. Fig. 15A shows the drive frequency and Fig. 15B shows the peak voltage in the longitudinal axes. The horizontal axes thereof are commonly the speed S, and scales in Figs. 15A and 15B are coincident.

As shown in Figs. 15A and 15B, the drive frequency increases in a speed range of $Sa \leq$ speed $S \leq S3'$ and a peak voltage increases in a speed range of $S3' \leq$ speed $S \leq Sb$. That is, unlike Embodiment 1, first, the resecting capability is improved through the change of the drive frequency, and when the drive frequency reaches the maximum value, the resecting capability is improved through the change of the peak voltage.

Sa and Sb in Embodiment 3 employ the same values as those employed in Embodiment 1. The reason is because Sa is the speed at which it is preferable to start improvement of the resecting capability and Embodiment 3 has in common with Embodiment 1 from this point of view. Sb is a lowest speed among speeds in which the drive frequency and the peak voltage are set to maximum values. Therefore, even if the order to be changed is switched, the value of Sb is the same as that in Embodiment 1. $S3'$ is a value which is determined as a speed when the drive frequency reaches the maximum value. Therefore, a value different from S3 in Embodiment 1 is employed for $S3'$. As a matter of course, Sa and Sb may be values different from those in Embodiment 1 and $S3'$ may be a value the same as S3.

In Embodiment 3, it is also possible to stabilize the depth of resection similarly to Embodiment 1. Which of the peak voltage or the drive frequency is to be preferentially changed is determined by selecting the one by which the depth of resection is more stabilized based on the characteristics of the piezoelectric element 135.

The invention is not limited to the embodiments, examples, or modification examples of the present specification and can be implemented by having various configurations within the scope not departing from the gist thereof. For example, technical features in the embodiments, examples, and modification examples corresponding to those in the aspects described in the summary section according to the invention can be appropriately replaced or combined in order to solve a part or all of the above-described problems or in order to achieve a part or all of the above-described effects. If the technical features are not described as being essential in the present specification, the technical features can be appropriately deleted. The following are examples thereof.

The drive frequency may not be changed. That is, adjustment of the resecting capability may be implemented through the change of the peak voltage and the supply flow rate. The adjustment of the resecting capability may also be implemented only through the change of the peak voltage without changing the drive frequency and the supply flow rate. Alternately, the adjustment of the resecting capability may also be implemented only through the change of the supply flow rate without changing the peak voltage and the drive frequency. It is possible to simplify the configuration of the control portion by employing a configuration in which the peak voltage and the drive frequency are not changed.

The peak voltage, the drive frequency, and the supply flow rate may be determined using a function. The speed range in which the peak voltage is changed and the speed range in which the drive frequency is changed may overlap each other. The drive waveform may not be a combination of sine curves, and for example, may be increased or decreased stepwise. The relationships between the peak voltage and the speed of the ejection port and between the drive frequency and the speed of the ejection port may be defined to be curvilinear or may be defined to be stepwise. The drive frequency may be changed while fixing the startup time. That is, the drive frequency may be changed by changing the time until a voltage of a driving signal reaches zero from a peak. Accordingly, it is possible to exclude the influence due to a change of the startup time when determining the drive frequency with respect to the movement speed, thereby easily determining the drive frequency.

For example, the speed of the ejection port may be calculated by an acceleration sensor provided at a tip end of the ejection port. It is considered that the calculation result becomes more accurate in this case. Alternately, the speed of the ejection port may be calculated using image processing. For example, the speed of the ejection port may be calculated by providing a marker at the tip end of the ejection port and by capturing the movement of the marker using a camera. When a robot operates a fluid ejecting apparatus, the robot can grasp the speed of the ejection port. Therefore, it is unnecessary to calculate the speed of the ejection port, and the grasped value may be used. The movement speed of a fluid ejection port may be calculated in consideration of the movement speed of a lesion portion. The measurement of the movement speed of the lesion portion may be implemented by predicting or measuring the movement caused by respiration or pulses. The subject of detection of the movement speed is not limited to the ejection port, and a site that moves along with the movement of the ejection port may be detected or the movement speed of the fluid ejection port may be detected.

In addition, the control of ejecting a fluid may be performed such that at least any one of a predetermined amount of a fluid, a predetermined energy of a fluid, and a predetermined pressure of a fluid is applied to a target to which the fluid is ejected regardless of the change of the movement speed of the fluid ejection port. The control may also be performed in a combination of two or more physical quantities among the predetermined amount of a fluid, predetermined energy of a fluid, and predetermined pressure of a fluid.

The type of acceleration sensor may be an electrostatic capacitance type or a heat detection type. In addition, the sensor is not limited to an acceleration sensor, and may be a sensor which can indirectly or directly detect the movement speed of the fluid ejection port. The fluid ejecting apparatus may be used in instruments other than medical instruments. For example, the fluid ejecting apparatus may be used in a cleaning apparatus that removes dirt using an ejected fluid. The fluid ejecting apparatus may be used in a drawing apparatus that draws a line or the like using an ejected fluid. The method of fluid ejection may use a laser beam. The fluid ejection system using a laser beam may intermittently irradiate a fluid with a laser beam and use pressure variation which occurs by evaporating a fluid, for example.

Embodiment 4

Embodiment 4 will be described.

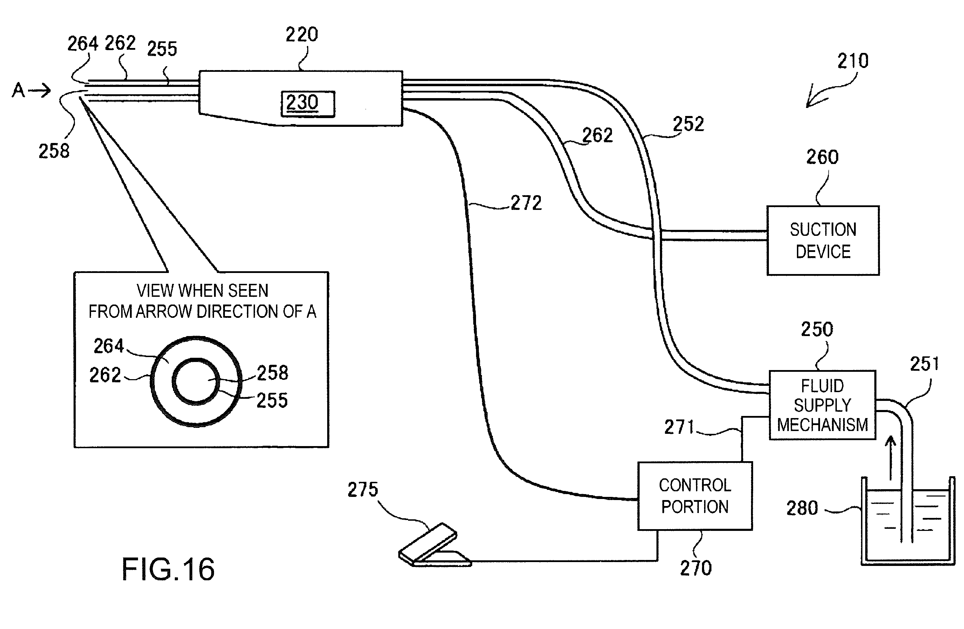

Fig. 16 is a configuration of a fluid ejecting apparatus 210. The fluid ejecting apparatus 210 is a medical instrument used in medical institutions and has a function of making an incision or resecting a lesion portion by ejecting a fluid to the lesion portion.

The fluid ejecting apparatus 210 includes a fluid ejection mechanism 220, a fluid supply mechanism 250, a suction device 260, a control portion 270, and a fluid container 280. The fluid supply mechanism 250 and the fluid container 280 are connected to each other through a connection tube 251. The fluid supply mechanism 250 and the fluid ejection mechanism 220 are connected to each other through a fluid supply passage 252. The connection tube 251 and the fluid supply passage 252 are formed of resin. The connection tube 251 and the fluid supply passage 252 may be formed of materials other than resin (for example, metal).

The fluid container 280 stores physiological saline. Pure water or fluid medicine may be used instead of the physiological saline. The fluid supply mechanism 250 supplies the fluid ejection mechanism 220 with a fluid, which is sucked from the fluid container 280 via the connection tube 251, via the fluid supply passage 252.

The fluid ejection mechanism 220 is an instrument, which is operated by a user by hand, of the fluid ejecting apparatus 210. The user performs discission or resection on a lesion portion by bringing a fluid, which is intermittently ejected from an ejection port 258, into contact with the lesion portion.

The control portion 270 transmits a driving signal to a pulsation generation portion 230 through a signal cable 272. The control portion 270 controls the flow rate of a fluid which is supplied to the pulsation generation portion 230 by controlling the fluid supply mechanism 250 through a control cable 271. A foot switch 275 is connected to the control portion 270. When a user turns on the foot switch 275, the control portion 270 performs supplying of a fluid to the pulsation generation portion 230 by controlling the fluid supply mechanism 250 and generates pulsation using the pressure of the fluid supplied to the pulsation generation portion 230 by transmitting the driving signal to the pulsation generation portion 230.

The suction device 260 is used for sucking a fluid or resected substance in the periphery of the ejection port 258. The suction device 260 and the fluid ejection mechanism 220 are connected to each other through a suction passage 262. The suction device 260 sucks the inside of the suction passage 262 at all times while a switch is turned on. The suction passage 262 is opened in the vicinity of a tip end of an ejection tube 255 by passing through the fluid ejection mechanism 220.

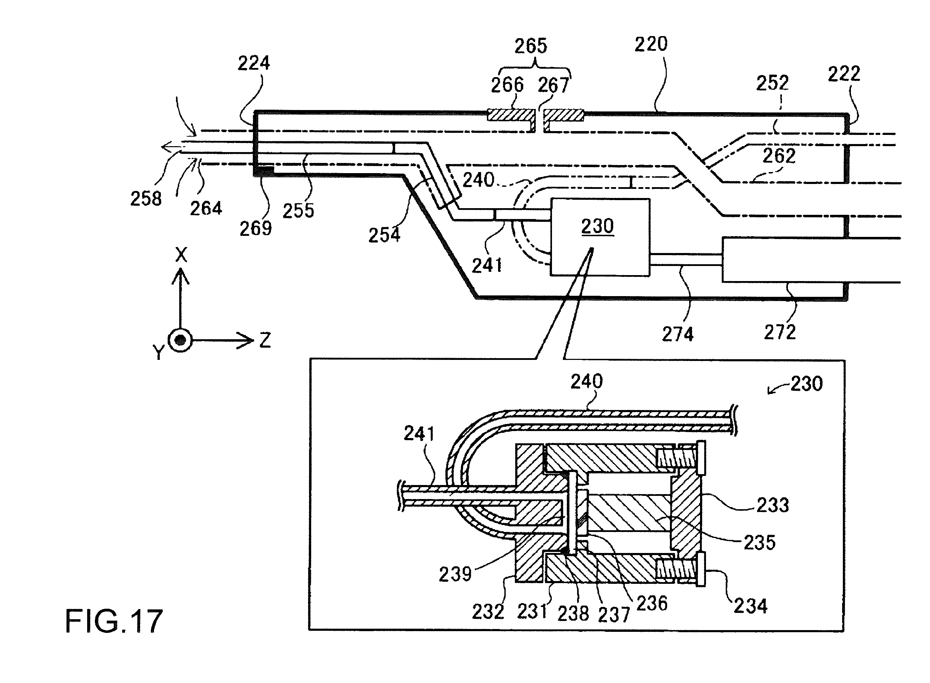

The suction passage 262 covers the ejection tube 255 in the fluid ejection mechanism 220. For this reason, as shown in a view when seen from the arrow direction of A of Fig. 16, the wall of the ejection tube 255 and the wall of the suction passage 262 form approximately concentric cylindrical shapes. A passage, through which a sucked substance which is sucked from a suction port 264 as a tip end of the suction passage 262 flows, is formed between the outer wall of the ejection tube 255 and the inner wall of the suction passage 262. The sucked substance is sucked into the suction device 260 through the suction passage 262. The suctioning is adjusted by a suction force adjustment mechanism to be described later along with Fig. 17.

Fig. 17 shows an internal structure of the fluid ejection mechanism 220. The fluid ejection mechanism 220 is equipped with the pulsation generation portion 230, an inlet passage 240, an exit passage 241, a connection tube 254, and an acceleration sensor 269 therein, and includes the suction force adjustment mechanism 265.

The pulsation generation portion 230 generates pulsation on the pressure of a fluid supplied from the fluid supply mechanism 250 to the fluid ejection mechanism 220 through the fluid supply passage 252. The fluid in which the pulsation of the pressure is generated is supplied to the ejection tube 255. The fluid supplied to the ejection tube 255 is intermittently ejected from the ejection port 258. The ejection tube 255 is formed of stainless steel. The ejection tube 255 may be formed of other materials, for example, other metals such as brass or reinforced plastics having rigidity at a predetermined level or higher.

As shown in a lower part of Fig. 17, the pulsation generation portion 230 includes a first case 231, a second case 232, a third case 233, a bolt 234, a piezoelectric element 235, a reinforcing plate 236, a diaphragm 237, a packing 238, the inlet passage 240, and the exit passage 241. The first case 231 and the second case 232 are connected in a manner of facing each other. The first case 231 is a cylindrical member. One end portion of the first case 231 is sealed by being fixed to the third case 233 using the bolt 234. The piezoelectric element 235 is disposed in a space which is formed inside the first case 231.

The piezoelectric element 235 is a laminated piezoelectric element. An end of the piezoelectric element 235 is fixed to the diaphragm 237 through the reinforcing plate 236. The other end of the piezoelectric element 235 is fixed to the third case 233. The diaphragm 237 is made of a metallic thin film, and the periphery thereof is fixed to the first case 231. A fluid chamber 239 is formed between the diaphragm 237 and the second case 232. The capacity of the fluid chamber 239 is changed by driving of the piezoelectric element 235.

The signal cable 272 is inserted from a rear end portion 222 of the fluid ejection mechanism 220. Two electrode wires 274 are accommodated in the signal cable 272 and are connected to the piezoelectric element 235 in the pulsation generation portion 230. The driving signal transmitted from the control portion 270 is transmitted to the piezoelectric element 235 through the electrode wires 274 in the signal cable 272. The piezoelectric element 235 expands and contracts based on the driving signal.

The inlet passage 240 into which a fluid flows is connected to the second case 232. The inlet passage 240 is bent in a U shape and extends toward the rear end portion 222 of the fluid ejection mechanism 220. The fluid supply passage 252 is connected to the inlet passage 240. The fluid which is supplied from the fluid supply mechanism 250 is supplied to the fluid chamber 239 through the fluid supply passage 252.

When the piezoelectric element 235 expands and contracts at a predetermined frequency, the diaphragm 237 vibrates. When the diaphragm 237 vibrates, the capacity of the fluid chamber 239 fluctuates and the pressure of the fluid in the fluid chamber is pulsated. The fluid that passes through the fluid chamber 239 flows out from the exit passage 241.

The exit passage 241 is connected to the second case 232. The ejection tube 255 is connected to the exit passage 241 through a metallic connection tube 254. The fluid flowing out to the exit passage 241 is ejected from the ejection port 258 through the connection tube 254 and the ejection tube 255.

The suction force adjustment mechanism 265 adjusts a force by which the suction passage 262 sucks a fluid or the like from the suction port 264. The suction force adjustment mechanism 265 includes an operation portion 266 and a hole 267. The hole 267 is a through hole that connects between the suction passage 262 and the operation portion 266. When a user opens and closes the hole 267 using a finger of a hand holding the fluid ejection mechanism 220, the amount of air flowing into the suction passage 262 through the hole 267 is adjusted depending on the opening and closing status. Accordingly, the suction force of the suction port 264 is adjusted. The adjustment of the suction force may be implemented through the control by the suction device 260.

The fluid ejection mechanism 220 includes the acceleration sensor 269. The acceleration sensor 269 is a piezo-resistance type triaxial acceleration sensor. The three axes are axes of X, Y, and Z shown in Fig. 17. The X-axis is parallel to the penetration direction of the hole 267 and the upward direction is a positive direction. The Z-axis is parallel to the long axis direction of the ejection tube 255 and a direction to which a fluid is ejected is set to be a negative direction. The Y-axis is defined by a right-hand system based on the X-axis and the Z-axis.

As shown in Fig. 17, the acceleration sensor 269 is disposed in the vicinity of a tip end portion 224. The measurement result is input into the control portion 270 through the signal line (not shown) and the signal cable 272.

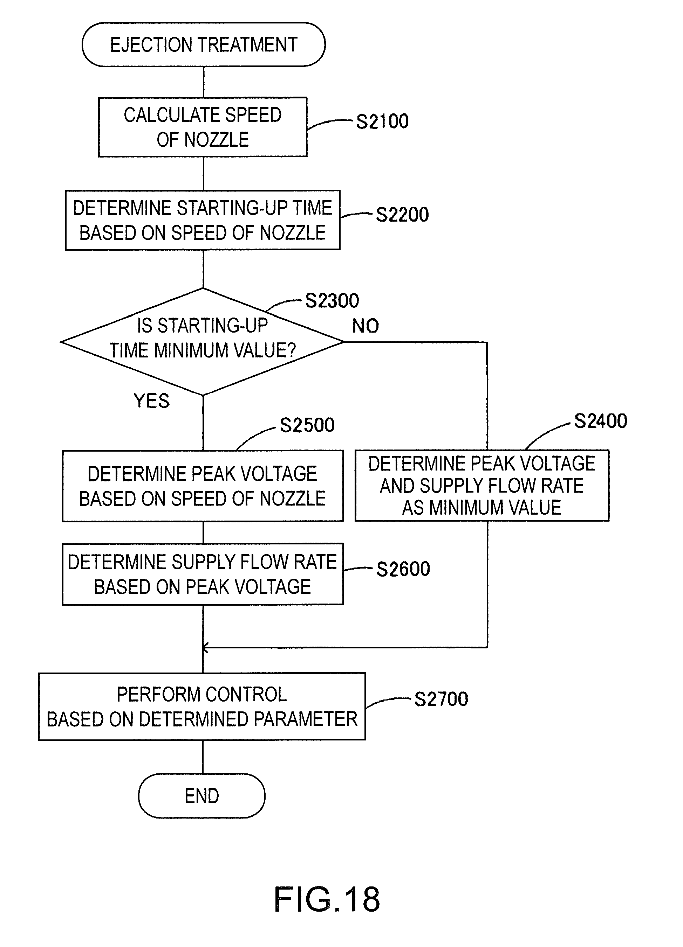

Fig. 18 is a flowchart showing ejection treatment. The ejection treatment is repeatedly performed by the control portion 270 while the foot switch 275 is stepped on. First, a speed S of the ejection port 258 is calculated (step S2100). The speed S referred herein is an absolute value of the speed in an XY plane, that is, an absolute value of the speed in which the speed in the Z-axis direction is neglected. The speed S is calculated based on the triaxial acceleration measured by the acceleration sensor 269.

The speed S is calculated as a parameter that affects the depth of resection of a lesion portion. This is because the resecting capability that acts on each local area of a lesion portion per unit time is affected by a relative speed between the ejection port 258 and the lesion portion. In the present embodiment, the speed S may be treated as the relative speed between the lesion portion and the ejection port 258 in consideration of a case where the lesion portion is moved in accordance with respiration of a patient or the like. However, it is described in the present embodiment on the assumption that the lesion portion maintains a condition having less than or equal to a predetermined movement amount.

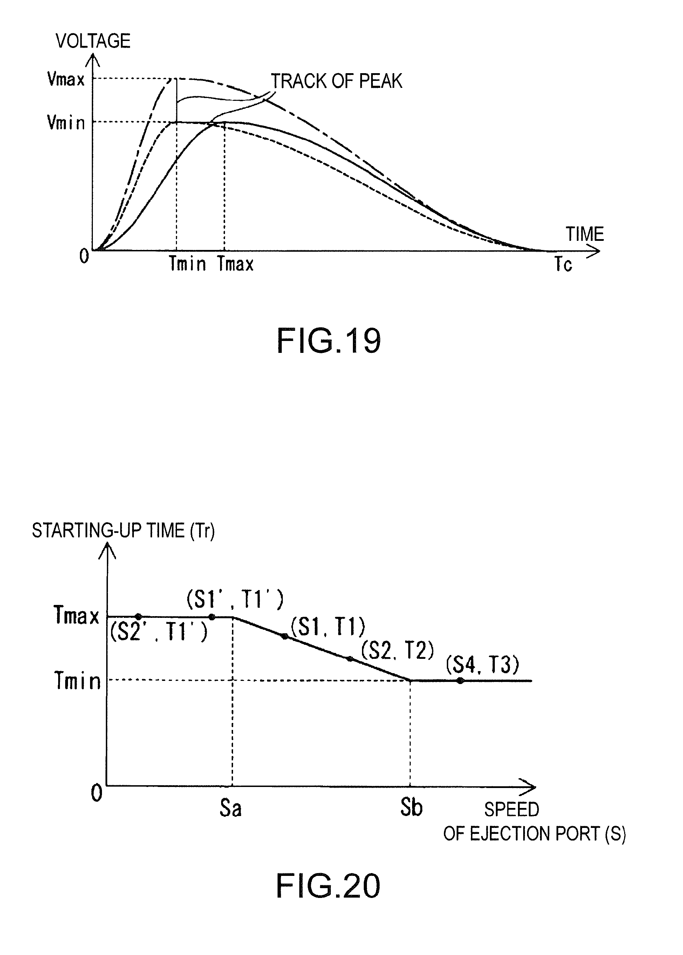

Subsequently, a startup time of a waveform of a driving signal (hereinafter, referred to as a "drive waveform") is determined based on the calculated speed S (step S2200). Fig. 19 is a graph showing a waveform of one period of drive waveforms. The longitudinal axis indicates voltage and the horizontal axis indicates time.

The drive waveforms of the present embodiment are described as a combination of sine curves. The waveform is expressed by the following expression until the voltage reaches a peak value from 0.

$$V(T) = Vp \{1 - \cos (\pi T / Tr)\} / 2 \text{ (where } 0 \leq T \leq Tr)$$

V is a voltage, Vp is a peak value of the voltage (peak voltage), T is a time, and Tr is a startup time. Vp is a variable value which is set within a range of Vmin ≤ Vp ≤ Vmax. Tr is a variable value which is set within a range of Tmin ≤ Tr ≤ Tmax. Vmax and Tmin are respectively values which are predetermined under conditions in which the load of the piezoelectric element 235 or the like is prevented from being excessively increased, and the like. Vmin and Tmax are respectively values which are predetermined under conditions in which the fluid is intermittently ejected, and the like. The peak voltage indicates a maximum voltage in one period of the drive waveforms used when ejecting the fluid.

The drive waveform is expressed by the following expression until the voltage reaches 0 from a peak value.

$$V(T) = Vp [1 + \cos \{\pi(T - Tr) / (Tc - Tr)\}] / 2 \text{ (where } Tr \leq T \leq Tc)$$

Tc is a time of one period of a drive waveform and is a fixed value in the present embodiment. As is clear from the above-described two expressions, the startup time Tr is a time until the voltage reaches the peak of the voltage from a predetermined voltage value in one period of the drive waveform.

When the voltage of the driving signal increases, the piezoelectric element 235 is deformed such that the fluid chamber 239 contracts in capacity. When the startup time Tr is shortened, the contraction of the fluid chamber 239 is performed over a short period of time. As a result, the fluid is vigorously ejected, and therefore, the resecting capability increases, and the depth of resection becomes deep.

Fig. 20 is a graph showing a relationship between the startup time Tr and the speed S in the present embodiment. As shown in Fig. 20, in a case of S ≤ Sa, the startup time Tr is fixed to Tmax. In a case of Sa ≤ S ≤ Sb, the startup time Tr is linearly decreases along with the increase of the speed S. In a case of S ≥ Sb, the startup time Tr is fixed to Tmin. In step S2200, the startup time Tr is determined in accordance with the relationship.

Subsequently, it is determined whether the startup time Tr is determined as a lower limit value (Tmin) (step S2300). In a case where the startup time Tr is determined as a value except for the lower limit value (step S2300: No), the peak voltage Vp and the supply flow rate are determined as minimum values (Vmin) (step S2400).

Meanwhile, when the startup time Tr is determined as the lower limit value (step S2300: Yes), the peak voltage Vp of the driving signal is determined based on the speed S (step S2500).

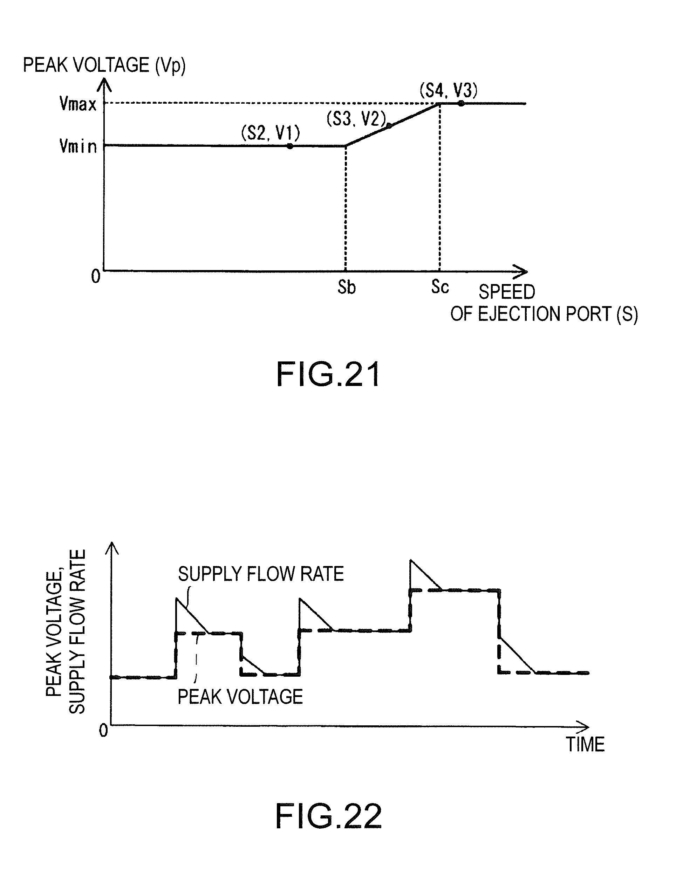

Fig. 21 is a graph showing a relationship between the peak voltage Vp and the speed S in the present embodiment. As shown in Fig. 21, in a case of S ≤ Sb, the peak voltage Vp is fixed to Vmin. In order to implement such a relationship, as described above, when the startup time Tr is not the lower limit value, the peak voltage Vp is fixed to Vmin.

In a case of Sb ≤ S ≤ Sc, the peak voltage Vp linearly increases along with the increase of the speed S. In a case of S ≥ Sc, the peak voltage Vp is fixed to Vmax. When performing step S2500, S is greater than or equal to Sb, and therefore, the peak voltage Vp is determined in accordance with the relationship with the peak voltage Vp in this speed region.

Since the startup time Tr and the peak voltage Vp are determined as described above, the peaks of the drive waveforms draw a track having an L-shape as shown in Fig. 19.

Subsequently, the supply flow rate is determined based on the peak voltage Vp (step S2600). Fig. 22 is a graph showing a technique of determining a flow rate. The longitudinal axis indicates a peak voltage Vp and a supply flow rate, and the horizontal axis indicates time. The change rate of the supply flow rate may be coincident with the change rate of the peak voltage. However, when the peak voltage is changed, the supply flow rate is made to be temporarily large.

For example, when the peak voltage becomes 2 x Vp1 in a state in which the peak voltage is Vp1 and the supply flow rate is F1, the supply flow rate is temporarily set to 3 x F1, and then, is gradually converged to 2 x F1. Alternately, when the peak voltage becomes 0.5 x Vp1 in a state in which the peak voltage is Vp1 and the supply flow rate is F1, the supply flow rate is temporarily set to 0.75 x F1, and then, is gradually converged to 0.5 x F1.

In this manner, abnormal ejection of a fluid due to an insufficient supply flow rate is avoided by making the supply flow rate temporarily large when the peak voltage is changed.

Finally, control is performed based on the determined parameters (startup time Tr, peak voltage Vp, and supply flow rate) (step S2700). As a result, a fluid is intermittently ejected from the ejection port 258 in accordance with the speed of the ejection port 258.

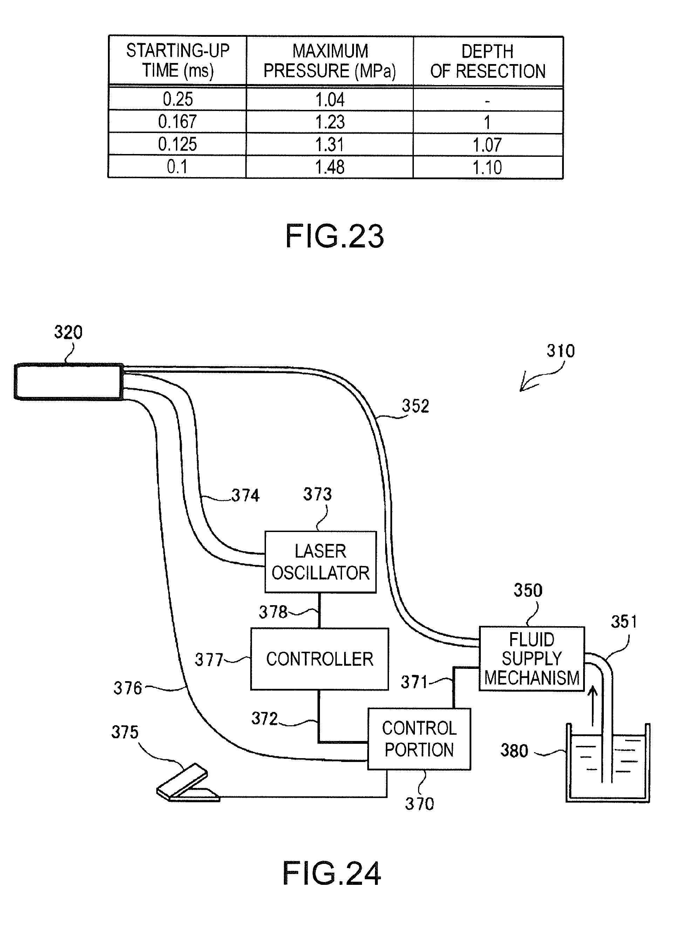

Fig. 23 is a table showing a test result in which the relationship between the startup time, a maximum pressure of an ejected fluid, and change of the depth of resection is examined. The depth of resection is based on a case in which the startup time is 0.375 milliseconds. The measurement of the depth of resection was performed under the same conditions except for the startup time. The test was performed without moving the ejection port 258.

As shown in Fig. 23, as the startup time becomes shorter, the maximum pressure of a fluid increases and the depth of resection becomes deep. Meanwhile, when the speed S is increased, the resecting capability that acts on each local area of a lesion portion is deteriorated. Accordingly, it is possible to stabilize the depth of resection by shortening the startup time when the speed S is increased.

Furthermore, according to the present embodiment, in a case in which the speed S is lower than or equal to Sb, excluded volume is not changed since the peak voltage Vp is constant. Therefore, it is unnecessary to change the supply flow rate, thereby facilitating the control.

The piezoelectric element 235 and the diaphragm 237 in the present embodiment are examples of variation portions in the appended claims. S1 to S4 shown in Figs. 20 and 21 are examples of first to fourth speeds, T1 to T3 shown in Fig. 20 are examples of a first to third times, V1 to V3 shown in Fig. 21 are examples of first to third voltages, S1' and S2' shown in Fig. 20 are examples of first and second predetermined times, and T1' shown in Fig. 20 is an example of a predetermined value.

The invention is not limited to the embodiments, examples, or modification examples of the present specification and can be implemented by having various configurations within the scope not departing from the gist thereof. For example, technical features in the embodiments, examples, and modification examples corresponding to those in the aspects described in the summary section of the invention can be appropriately replaced or combined in order to solve a part or all of the above-described problems or in order to achieve a part or all of the above-described effects. If the technical features are not described as being essential in the present specification, the technical features can be appropriately deleted. The following are examples thereof.

The startup time and the peak voltage may be determined using a function, and may be determined by substituting the speed S in a map which is mapped in advance. The treatment load decreases according to the map control.

The speed range in which the startup time is changed and the speed range in which the peak voltage is changed may overlap each other.

The drive waveform may not be a combination of sine curves, and for example, may be increased or decreased stepwisely.

The relationship between the startup time and the speed of the ejection port may be defined to be curvilinear or may be defined to be stepwise.

The definition of the startup time may not be a time until a driving signal reaches a peak from 0, and for example, may be a time until a driving signal reaches a value slightly smaller than the peak from a value slightly larger than 0.

The startup time may be defined as a time until a voltage reaches a value smaller than a certain voltage value from the certain voltage value in a configuration in which the volume of a fluid chamber contracts when the voltage of a driving signal decreases.

For example, the speed of the ejection port may be calculated by an acceleration sensor provided at a tip end of the ejection port. It is considered that the calculation result becomes more accurate in this case.

Alternately, the speed of the ejection port may be calculated using image processing. For example, the speed of the ejection port may be calculated by providing a marker at the tip end of the ejection port and by capturing the movement of the marker using a camera.

When a robot operates a fluid ejecting apparatus, the robot can grasp the speed of the ejection port. Therefore, it is unnecessary to calculate the speed of the ejection port, and the grasped value may be used.

The movement speed of a fluid ejection port may be calculated in consideration of the movement speed of a lesion portion. The measurement of the movement speed of the lesion portion may be implemented by predicting or measuring the movement caused by respiration or pulses.

In addition, energy which is provided to a fluid within a fluid chamber may be adjusted in accordance with the speed of an ejection port such that the fluid ejected from a fluid ejection mechanism is provided with the same energy per unit area of an ejection target.

In the embodiment, the fluid ejection mechanism 220 was described as an instrument which is operated by being held by hand of a user. However, the fluid ejection mechanism may be an instrument which is operated by being inserted into a living body as a fluid ejection mechanism used in an endoscope such as a laparoscope.

The type of acceleration sensor may be an electrostatic capacitance type or a heat detection type. In addition, the sensor is not limited to an acceleration sensor, and may be a sensor which can indirectly or directly detect the speed of the fluid ejection port.

The fluid ejecting apparatus may be used in instruments other than medical instruments.

For example, the fluid ejecting apparatus may be used in a cleaning apparatus that removes dirt using an ejected fluid.

The fluid ejecting apparatus may be used in a drawing apparatus that draws a line or the like using an ejected fluid.

Embodiment 5

Embodiment 5 will be described.

In Fig. 24, the fluid ejecting apparatus 310 includes a fluid ejection mechanism 320, a fluid supply mechanism 350, a control portion 370, a laser oscillator 373, a controller 377, and a fluid container 380. The fluid supply mechanism 350 and the fluid container 380 are connected to each other through a connection tube 351. The fluid supply mechanism 350 and the fluid ejection mechanism 320 are connected to each other through a fluid supply passage 352. The connection tube 351 and the fluid supply passage 352 are formed of resin. The connection tube 351 and the fluid supply passage 352 may be formed of materials other than resin (for example, metal).

The fluid container 380 stores physiological saline. Pure water or fluid medicine may be used instead of the physiological saline. The fluid supply mechanism 350 supplies the fluid ejection mechanism 320 with a fluid, which is sucked from the fluid container 380 via the connection tube 351, via the fluid supply passage 352 by driving a built-in pump.

The fluid ejection mechanism 320 is an instrument, which is operated by being held by hand of a user, of the fluid ejecting apparatus 310. The user performs discission or resection on a lesion portion by bringing a fluid, which is intermittently ejected from the fluid ejection mechanism 320, into contact with the lesion portion.

The control portion 370 controls the flow rate (hereinafter, referred to as "supply flow rate") of a fluid which is supplied to the fluid ejection mechanism 320 by controlling the fluid supply mechanism 350 through a control cable 371. A foot switch 375 is connected to the control portion 370. When a user turns on the foot switch 375, the control portion 370 performs supplying of a fluid to the fluid ejection mechanism 320 by controlling the fluid supply mechanism 350 and transmits a driving signal to the controller 377 through the signal cable 372.

The controller 377 outputs a control signal through the signal cable 378 in order to cause the laser oscillator 373 to output a laser beam corresponding to the driving signal. The laser oscillator 373 is formed by a holmum YAG laser and outputs a laser beam according to the control signal. The wavelength of the laser beam is 2.06 μm. The output laser beam is introduced to the fluid ejection mechanism 320 through a cable 374 for the laser which is formed of optical fibers.

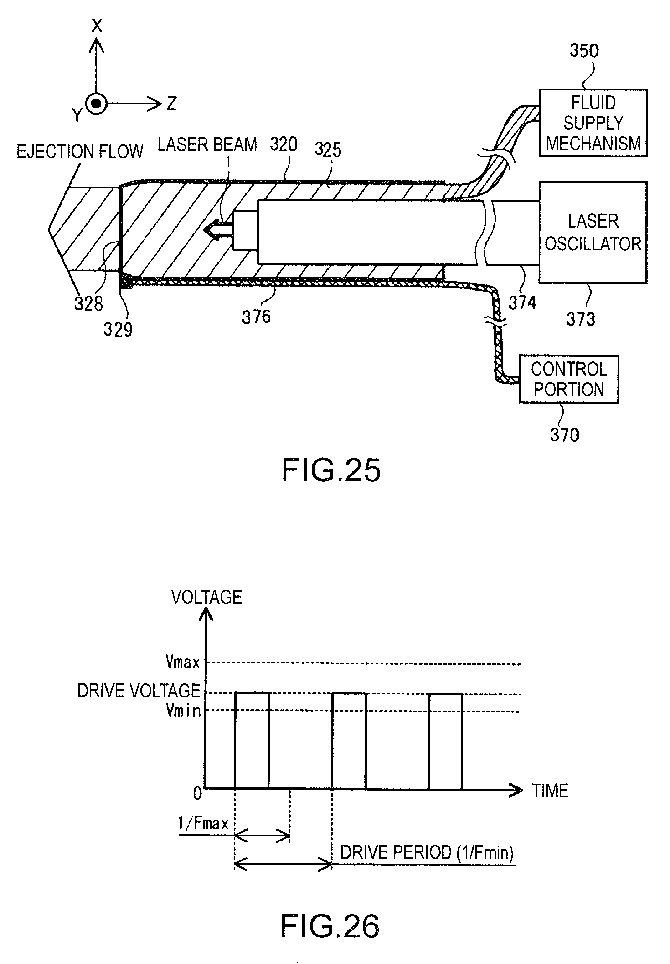

Fig. 25 is a cross-sectional view showing the inside of a fluid ejection mechanism 320. A fluid chamber 325 is formed in the fluid ejection mechanism 320. The fluid chamber 325 is filled with a fluid which is supplied from the fluid supply mechanism 350. The laser beam introduced by the cable 374 for the laser is released in the fluid ejection mechanism 320. The released laser beam is absorbed into the fluid filled in the fluid ejection mechanism 320. The fluid absorbing the laser beam is evaporated by the energy of the absorbed laser beam. In the present embodiment, the output of the laser beam is intermittently performed, and therefore, the evaporation also occurs intermittently. The evaporation occurring intermittently instantly makes the pressure of the fluid in the fluid ejection mechanism 320 large. The pressure which is instantly made large causes the fluid to be ejected from the ejection port 328.

The fluid ejection mechanism 320 includes an acceleration sensor 329. The acceleration sensor 329 is a piezo-resistance type triaxial acceleration sensor. As shown in Fig. 25, the acceleration sensor 329 is disposed in the vicinity of the ejection port 328 and outside the housing of the fluid ejection mechanism 320. The measurement result is input to the control portion 370 through a cable 376 for the acceleration sensor. The cable 376 for the acceleration sensor is bonded to the outside of the housing of the fluid ejection mechanism 320 from a connection portion of the cable 376 for the acceleration sensor to a rear end (on the side opposite to the ejection port 328) of the fluid ejection mechanism 320.

The three axes to be measured by the acceleration sensor 329 are axes of X, Y, and Z shown in Fig. 25. The Z-axis is parallel to the long axis direction of the fluid ejection mechanism 320, that is, parallel to the ejection direction of a fluid, and the direction to which a fluid is ejected is set to be a negative direction. The X-axis is orthogonal to the Z-axis, and a predetermined direction is set to a positive direction. The predetermined direction is an upward direction in a vertical direction when the Z-axis is made horizontal and the acceleration sensor 329 is positioned immediately below the Z-axis as shown in Fig. 25. The Y-axis is defined by a right-hand system based on the X-axis and the Z-axis.

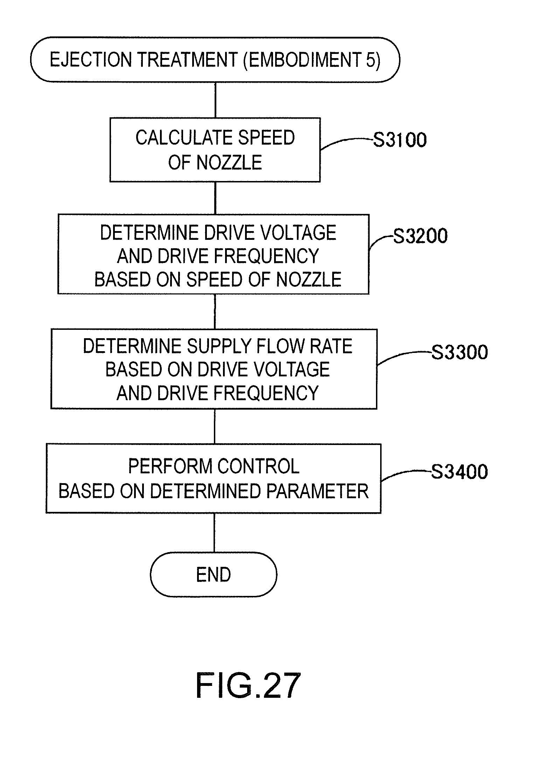

Fig. 26 is a graph showing a waveform of a driving signal. The driving signal is input to the controller 377 in order to output the laser beam as described above. The longitudinal axis indicates voltage and the horizontal axis indicates time. The waveform of the driving signal in the present embodiment is a pulse wave as shown in Fig. 26. The maximum voltage (hereinafter, referred to as a "drive voltage") of each pulse wave and the frequency (hereinafter, referred to as a "drive frequency") of the pulse wave are changed by ejection treatment (which will be described along with Fig. 27).

Fig. 26 exemplifies a case in which the drive voltage is a value between a maximum value (Vmax) and a minimum value (Vmin) and the drive period is a maximum value, that is, the drive frequency is a minimum value (Fmin). If the drive voltage is large, the controller 377 controls the laser oscillator 373 such that the energy of the laser beam (pulsed light) which is released at once increases. Meanwhile, if the drive frequency is large, the controller 377 controls the laser oscillator 373 such that the number of times of releasing the laser beam per unit time increases. In both cases, the output (energy per unit time) of the released laser beam becomes large. When the output of the laser beam becomes large in this manner, the resecting capability is improved as will be described below.

Fig. 27 is a flowchart showing ejection treatment. The ejection treatment is repeatedly performed by the control portion 370 while the foot switch 375 is stepped on. First, a speed S of the ejection port 328 is calculated (step S3100) . The speed S referred to herein is an absolute value of the speed in an XY plane, that is, an absolute value of the speed in which the speed in the Z-axis direction is neglected. The speed S is calculated based on the triaxial acceleration measured by the acceleration sensor 329.

The speed S is calculated as a parameter that affects the depth of resection of a lesion portion. This is because the resecting capability that acts on each local area of a lesion portion per unit time is affected by a relative speed between the ejection port 328 and the lesion portion. Accordingly, the speed S may be treated as a movement speed between the lesion portion and the ejection port 328 in consideration of a case in which the lesion portion is moved due to respiration of a patient or the like. However, in the present embodiment, the speed S is treated as a movement speed between the lesion portion and the ejection port 328 based on the assumption that the lesion portion is stationary.

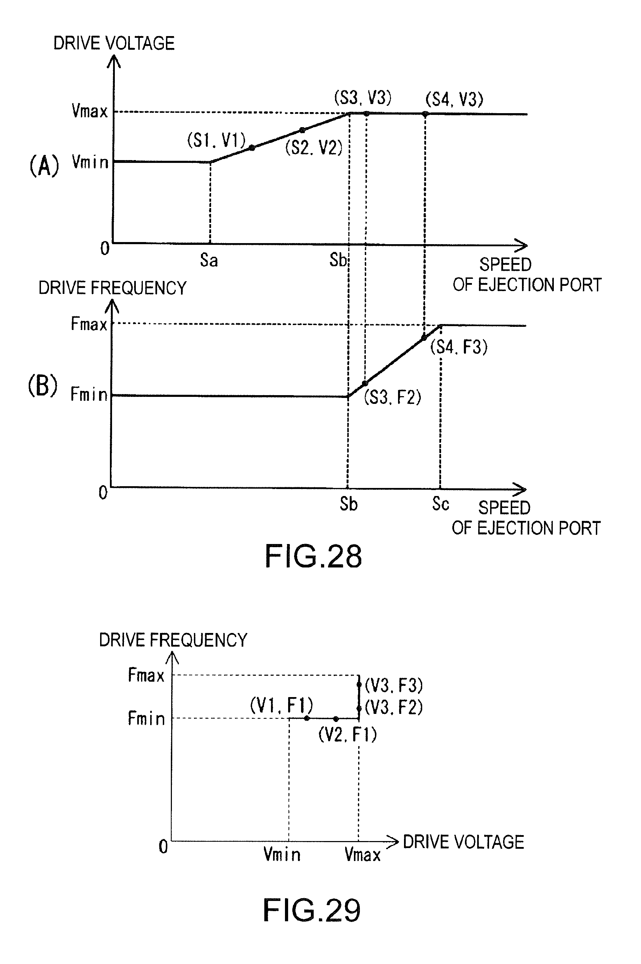

Subsequently, a drive voltage and a drive frequency are determined based on the calculated speed S (step S3200). Figs. 28A and 28B are graphs showing relationships between the drive voltage and the speed S and between the drive frequency and the speed S. Fig. 28A shows the drive voltage and Fig. 28B shows the drive frequency in the longitudinal axes. The horizontal axes thereof are commonly the speed S, and scales in Figs. 28A and 28B are coincident.

As shown in Figs. 28A and 28B, in each of speed ranges which are Sa ≤ speed S ≤ Sb and Sb ≤ speed S ≤ Sc, the parameters in which the values change are different from each other. That is, Sa, Sb, and Sc are speeds determined in advance as a threshold value for switching the parameters to be changed.

In a case of speed S ≤ Sa, the drive voltage is fixed to Vmin which is a minimum value and the drive frequency is fixed to Fmin which is a minimum value. In a case where the parameters are set in this manner, the resecting capability becomes lowest.

In a case of Sa ≤ speed S ≤ Sb, the drive voltage linearly increases with respect to the increase of the speed S while the drive frequency is fixed to Fmin. In a case of speed S = Sb, the drive voltage is set to Vmax which is a maximum value. Vmin is set such that the resecting capability is prevented from being excessively decreased. Vmax is set such that the load of the laser oscillator 373 is prevented from being excessively increased. If the drive voltage increases, energy of a laser beam which is released at once increases and the pressure variation inside of the fluid ejection mechanism 320 increases. As a result, the fluid is vigorously ejected and the resecting capability increases. Therefore, the depth of resection is stabilized even if the speed S is fast.

In a case of Sb ≤ speed S ≤ Sc, the drive frequency linearly increases with respect to the increase of the speed S while the drive voltage is fixed to Vmax. In a case of speed S = Sc, the drive frequency is set to Fmax which is a maximum value. Fmin is set such that the resecting capability is prevented from being excessively decreased. Fmax is set such that the load of the laser oscillator 373 is prevented from being excessively increased. If the drive frequency increases, the number of times of ejecting a fluid per unit time increases. As a result, the resecting capability increases, and the depth of resection is stabilized even if the speed S is fast.

The supply flow rate is determined after the drive voltage and the drive frequency are determined in this manner (step S3300). The supply flow rate is determined so as to be a value which is sufficient to replenish a fluid which is intermittently ejected. In both the cases of the drive voltage and the drive frequency, if the value increases, the amount of fluid ejected per unit time increases. Accordingly, in a case in which at least one of the drive voltage and the drive frequency is set to be large, the supply flow rate is set to be large. Finally, control is performed based on the determined drive voltage, drive frequency, and supply flow rate (step S3400).

As described above, it is possible to change the energy of the laser beam output at once and to stabilize the depth of resection by changing the drive voltage such that the resecting capability is improved along with the increase of the speed S. The energy of the laser beam output at once has a relatively wide control width, and therefore, is preferable as a parameter for controlling the output of the laser beam. Furthermore, it is possible to stabilize the depth of resection by changing the drive frequency when the drive voltage reaches a maximum value.

Fig. 29 is a graph showing a relationship between a drive frequency and a drive voltage. As described above, the drive frequency does not change within the range (Sa ≤ speed S ≤ Sb) of the speed S at which the drive voltage changes, but changes within the range (Sb ≤ speed S ≤Sc) of the speed S at which the drive voltage is Vmax. Since the ranges of the speed S at which the drive voltage and the drive frequency change are separated from each other in this manner, it is easy to determine the values of the drive voltage and the drive frequency in each of the speed ranges.

S1 to S4 shown in Figs. 28A, 28B and 29 are examples of first to fourth speeds in the appended claims, V1 to V3 shown in Figs. 28A to 29 are examples of first to third voltages in the appended claims, and F1 to F3 shown in Figs. 28A to 29 are examples of first to third frequencies in the appended claims. The laser oscillator 373 and the controller 377 in the embodiment are examples of output portions in the appended claims.

Embodiment 6

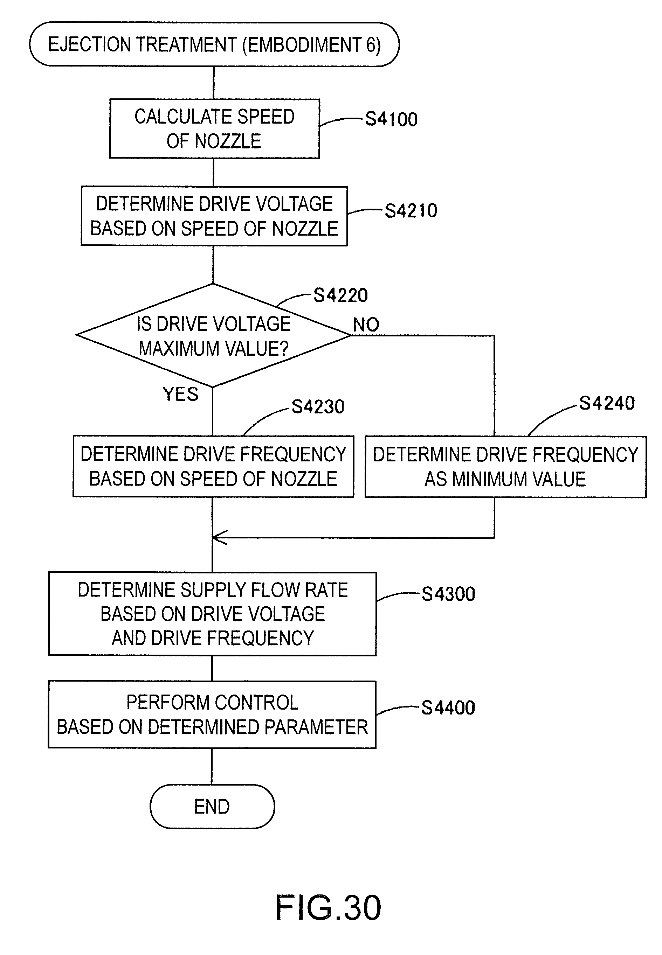

Embodiment 6 will be described. In Embodiment 6, ejection treatment shown in Fig. 30 will be performed instead of the ejection treatment shown in Fig. 27. The description of the hardware configuration the same as that in Embodiment 5 will be omitted. The description of steps S4100, S4300, and S4400 in the ejection treatment of Embodiment 6 which are the same as steps S3100, S3300, and S3400 in Embodiment 5 will be omitted. In Embodiment 6, steps S4210 to S4240 are performed instead of step S3200 of Embodiment 5.

After calculating the speed S (step S4100), the drive voltage is determined based on the calculated speed S (step S4210). The method of determining the drive voltage is the same as that in Embodiment 5. In a case of speed S ≤ Sa, the drive voltage is fixed to Vmin, in a case of Sa ≤ speed S ≤ Sb, the drive voltage linearly increases in accordance with the increase of the speed S, and in a case of Sb ≤ speed S, the drive voltage is fixed to Vmax.

Next, it is determined whether the drive voltage is set to a maximum value (Vmax) (step S4220). When the drive voltage is set to a value lower than the maximum value (step S4220: No), the drive frequency is set to a minimum value (Fmin) (step S4240). The setting of the drive voltage to the value lower than the maximum value means that there is a room for improvement in the resecting capability through the change of the drive voltage. Accordingly, it is unnecessary to improve the resecting capability by changing the value of the drive frequency, and therefore, the drive frequency is set to a minimum value.

On the other hand, when the drive voltage is set to a maximum value (step S4220: Yes), the drive frequency is determined based on the speed S (step S4230). The method of determining the drive frequency is the same as that in Embodiment 5. In a case of speed S ≤ Sb, the drive frequency is fixed to Fmin, in a case of Sb ≤ speed S ≤ Sc, the drive frequency linearly increases in accordance with the increase of the speed S, and in a case of Sc ≤ speed S, the drive frequency is fixed to Fmax.

The setting of the drive voltage to the maximum value means that there is no room for improvement in the resecting capability through the change of the drive voltage. Therefore, step S4230 is performed in order to improve the resecting capability by changing the value of the drive frequency. In Embodiment 6, it is also possible to obtain the control result the same as that in Embodiment 5.

The invention is not limited to the embodiments, examples, or modification examples of the present specification and can be implemented by having various configurations within the scope not departing from the gist thereof. For example, technical features in the embodiments, examples, and modification examples corresponding to those in the aspects described in the summary section of the invention can be appropriately replaced or combined in order to solve a part or all of the above-described problems or in order to achieve a part or all of the above-described effects. If the technical features are not described as being essential in the present specification, the technical features can be appropriately deleted. The following are examples thereof.

The drive voltage and the drive frequency may be determined using a function. The speed range in which the drive voltage is changed and the speed range in which the drive frequency is changed may overlap each other. The waveform of a driving signal may not be a pulse wave and may be a sine curve or the like, for example. The relationships between the drive voltage and the speed of the ejection port and between the drive frequency and the speed of the ejection port may be defined to be curvilinear or may be defined to be stepwise. Only one of the drive voltage and the drive frequency may be changed. When changing the drive voltage, a voltage at greater than or equal to a predetermined value or a voltage for a predetermined period may be changed without being limited to the change of the maximum voltage.

At least any one of the drive voltage and the drive frequency may be changed in accordance with the distance between an ejection port and a lesion portion. It is because it is considered that the distance between the ejection port and the lesion portion is a parameter relating to the depth of resection similar to the movement speed between the ejection port and the lesion portion. Specifically, at least any one of the drive voltage and the drive frequency may be changed such that the resecting capability is improved in accordance with the distance between the ejection port and the lesion portion being separated. The output of a laser beam may be adjusted by changing the pulse width. The pulse width is a time at which a driving signal reaches a maximum voltage.

The speed of the ejection port may be calculated using image processing. For example, the speed of the ejection port may be calculated by providing a marker in the vicinity of the ejection port and by capturing the movement of the marker using a camera. When a robot operates a fluid ejecting apparatus, the robot can obtain the speed of the ejection port based on calculation of travelled distance and moving speed of the ejection port or the robot arm having the ejection port. The robot can obtain the moving speed of the ejection port or the robot arm when a moving speed can be selected from a plurality of predetermined preset moving speeds. Therefore, it can be unnecessary to calculate the speed of the ejection port, and the grasped value may be used. The movement speed of a fluid ejection port may be calculated in consideration of the movement speed of a lesion portion. The measurement of the movement speed of the lesion portion may be implemented by predicting or measuring the movement caused by respiration or pulses. The subject of detection of the movement speed is not limited to the ejection tube and a site that moves along with the movement of the ejection tube may be detected or the movement speed of the fluid ejection port may be detected.

The type of acceleration sensor may be an electrostatic capacitance type or a heat detection type. In addition, the sensor is not limited to an acceleration sensor, and may be a sensor which can indirectly or directly detect the speed of the fluid ejection port. The fluid ejecting apparatus may be used in instruments other than medical instruments. For example, the fluid ejecting apparatus may be used in a cleaning apparatus that removes dirt using an ejected fluid. The fluid ejecting apparatus may be used in a drawing apparatus that draws a line or the like using an ejected fluid.

The type of laser may be a solid laser other than the holmum YAG laser, or may be a semiconductor laser, a fluid laser, and a gas laser. When changing the type of fluids to be ejected, the wavelength of a laser beam may be changed to a wavelength likely to be absorbed by the changed fluid. The method of supplying the fluid may not use driving of a pump, and may use the weight of the fluid itself, for example, an instillation device.

**Claims**

1.  A robotic surgical apparatus provided with a robot arm portion, an operation portion (166, 266) that receives an input by a user for operating the robot arm portion, a tool control portion configured to operate the robot arm portion based on the input which the operation portion (166, 266) receives from a user, and a fluid ejecting portion configured to eject a fluid jet from a nozzle thereof, the fluid ejecting portion comprising:

    a pulsating flow-provision portion including a fluid chamber (139, 239, 325) and configured to provide a fluid within the fluid chamber (139, 239, 325) with a pulsating flow;
    a fluid ejection tube (155, 255) which includes a fluid ejection port (158, 258, 328) for ejecting a fluid;
    a fluid supply portion (150, 250, 350) which supplies a fluid to the fluid chamber (139, 239, 325); and
    a fluid ejection control portion which controls the pulsating flow-provision portion,
    wherein the fluid ejection tube (155, 255) is mechanically fixed to the robot arm portion, and the fluid ejection control portion is configured such that the ejection amount of a fluid per unit time can be changed in accordance with the movement speed (S) of the fluid ejection port (158, 258).

2.  The robotic surgical apparatus according to claim 1, wherein the pulsating flow-provision portion includes a capacity variation portion (135, 137, 235, 237) that changes the capacity in the fluid chamber (139, 239), wherein the robotic surgical apparatus further comprises

    - a fluid supply portion (150, 250) which supplies a fluid to the fluid chamber (139, 239); and
    - a control portion (170, 270) which controls the capacity variation portion (135, 137, 235, 237) and the fluid supply portion (150, 250), and

    wherein the control portion (170, 270) changes at least one of a voltage applied to the capacity variation portion (135, 137, 235, 237) and a flow rate of a fluid supplied to the fluid chamber (139, 239) in accordance with the movement speed of the fluid ejection port (158, 258).

3.  The robotic surgical apparatus according to claim 2, wherein the control portion (170, 270) sets the voltage to a first voltage (V1) when the movement speed (S) of the fluid ejection port (158, 258) is a first speed, and sets the voltage to a second voltage (V2) higher than the first voltage (V1) when the movement speed (S) is a second speed (S2) faster than the first speed (S1).

4.  The robotic surgical apparatus according to claim 2 or 3, wherein the control portion (170, 270) sets the flow rate of the fluid supplied to the fluid chamber (139, 239, 325) to a first flow rate when the movement speed (S) is a first speed (S1), and sets the flow rate of the fluid supplied to the fluid chamber (139, 239, 325) to a second flow rate larger than the first flow rate when the movement speed (S) is a second speed (S2).

5.  The robotic surgical apparatus according to any one of claims 2 to 4,
    wherein a driving signal is applied to the capacity variation portion (135, 137, 235, 237), and
    wherein the control portion (170, 270) changes the voltage at the movement speed (S) lower than or equal to a third speed (S3) which is faster than a second speed (S2), sets the frequency of the driving signal to a first frequency (F1) when the movement speed (S) is the third speed (S3), and sets the frequency of the driving signal to a second frequency (F2) which is higher than the first frequency (F1) when the movement speed (S) is a fourth speed (S4) which is faster than the third speed (S3).

6.  The robotic surgical apparatus according to any one of claims 2 to 5, wherein the control portion (170, 270) performs control of the voltage, the flow rate, and a frequency of the driving signal in accordance with the movement speed (S).

7.  The robotic surgical apparatus according to claim 1, wherein

the pulsating flow-provision portion includes a pressurization portion (373) that pressurizes the inside of the fluid chamber (325); and
the robotic surgical apparatus comprises a controller (377) that changes a driving signal transmitted to the pressurization portion (373) in accordance with the movement speed of the fluid ejection port.

8. The robotic surgical apparatus according to claim 7, wherein the controller (377) changes a time until the driving signal reaches a second predetermined voltage (V2) from a first predetermined voltage (V1) which is smaller than the second predetermined voltage in accordance with the movement speed (S) of the fluid ejection port (158, 258).

9. The robotic surgical apparatus according to claim 7 or 8, wherein the controller (377) sets a startup time to a first time when the movement speed (S) is a first speed (S1), and sets the startup time to a second time shorter than the first time when the movement speed (S) is a second speed (S2) faster than the first speed (S1).

10. The robotic surgical apparatus according to claim 7, 8 or 9, wherein the controller (377) sets a maximum voltage of the driving signal to a first voltage (V1) when the movement speed (S) is a second speed (S2), and sets the maximum voltage of the driving signal to a second voltage (V2) higher than the first voltage (V1) when the movement speed (S) is a third speed (S3) faster than the second speed (S2).

11. The robotic surgical apparatus according to any one of claims 7 through 10, wherein the controller (377) sets the flow rate of the fluid to a first flow rate when the movement speed (S) is a second speed (S2), and sets the flow rate of the fluid to a second flow rate larger than the first flow rate when the movement speed (S) is a third speed (S3) which is larger than the second speed (S2).

12. The robotic surgical apparatus according to any one of claims 7 through 11, wherein, when the movement speed (S) is a fourth speed (S4) faster than a third speed (S3), the controller (377) sets a startup time to a third time shorter than a second time and sets a maximum voltage of the driving signal to a third voltage (V3) higher than a second voltage (V2).

13. The robotic surgical apparatus according to any one of claims 7 through 12, wherein the controller (377) sets the flow rate of the fluid to a third flow rate when the movement speed (S) is a fourth speed (S4) which is larger than the third speed.

14. The robotic surgical apparatus according to any one of claims 7 through 13, wherein, when the movement speed (S) is a first predetermined speed slower than a first speed (S1) and is a second predetermined speed slower than the first predetermined speed, the controller (377) sets a startup time, the maximum voltage of the driving signal, and the flow rate of the fluid to predetermined values respectively.

15. A fluid ejecting apparatus (110) for a robotic surgical apparatus, the fluid ejecting apparatus (110) comprising:

a pulsating flow-provision portion including a fluid chamber (139, 239, 325) and configured to provide a fluid within the fluid chamber (139, 239, 325) with a pulsating flow;
a fluid ejection tube (155, 255) which includes a fluid ejection port (158, 258, 328) for ejecting a fluid; and
a fluid ejection control portion which controls the pulsating flow-provision portion,
wherein the fluid ejection tube (155, 255) can be mechanically fixed to a robot arm portion of the robotic surgical apparatus, and
wherein the fluid ejection control portion is configured such that the ejection amount of a fluid per unit time can be changed in accordance with the movement speed (S) of the fluid ejection port (158, 258, 328).

FIG. 1

FIG. 2

FIG. 3

VIEW WHEN SEEN
FROM ARROW DIRECTION OF A

SUCTION
DEVICE

FLUID
SUPPLY
MECHANISM

CONTROL
PORTION

EP 2 873 387 A1

FIG. 4

EP 2 873 387 A1

VOLTAGE

PEAK
VOLTAGE

0

1/FREQUENCY | 1/FREQUENCY | 1/FREQUENCY

TIME

## FIG. 5

EJECTION TREATMEMT (EMBODIMENT 1)

CALCULATE SPEED
OF NOZZLE ———S1100

DETERMINE PEAK VOLTAGE
AND DRIVE FREQUENCY BASED ON SPEED
OF NOZZLE ———S1200

DETERMINE SUPPLY FLOW RATE
BASED ON PEAK VOLTAGE
AND DRIVE FREQUENCY ———S1300

PERFORM CONTROL
BASED ON DETERMINED PARAMETER ———S1400

END

## FIG. 6

PEAK VOLTAGE

(A)

Vmax

Vmin

0            Sa              S3           SPEED
                                         OF EJECTION PORT

(S1, V1)    (S2, V2)

DRIVE FREQUENCY

(B)

Fmax

Fmin

0                      S3        Sb    SPEED
                                       OF EJECTION PORT

(S4, F2)

(S3, F1)

FIG. 7

VOLTAGE

TRACK OF PEAK POINTS

Vmax

Vmin

B (IN CASE OF SPEED S = S3)

J (IN CASE OF SPEED S ≤ Sa)

C
( IN CASE OF
 SPEED S ≥ Sb )

0              1/Fmax           1/Fmin    TIME

FIG. 8

26

FIG. 9

FIG.10

FIG.11

FIG.12

FIG.13

EJECTION TREATMEMT (EMBODIMENT 2)

CALCULATE SPEED OF NOZZLE —S1100

DETERMINE PEAK VOLTAGE BASED ON SPEED OF NOZZLE —S1210

—S1220
IS PEAK VOLTAGE MAXIMUM VALUE?　　NO

YES

—S1230
DETERMINE DRIVE FREQUENCY BASED ON SPEED OF NOZZLE

—S1240
DETERMINE DRIVE FREQUENCY AS MINIMUM VALUE

DETERMINE SUPPLY FLOW RATE BASED ON PEAK VOLTAGE AND DRIVE FREQUENCY —S1300

PERFORM CONTROL BASED ON DETERMINED PARAMETER —S1400

END

FIG.14

FIG.15

FIG.16

VIEW WHEN SEEN
FROM ARROW DIRECTION OF A

264
262
258
255

220
230
210
264 262 255
A→
258
252
262
272
260
SUCTION
DEVICE
250
251
FLUID
SUPPLY
MECHANISM
271
275
CONTROL
PORTION
280
270

EP 2 873 387 A1

FIG.17

EJECTION TREATMENT

CALCULATE SPEED
OF NOZZLE ——S2100

DETERMINE STARTING-UP TIME
BASED ON SPEED OF NOZZLE ——S2200

——S2300

IS STARTING-UP
TIME MINIMUM VALUE?                    NO

YES

——S2500                                          ——S2400

DETERMINE PEAK VOLTAGE
BASED ON SPEED OF NOZZLE

DETERMINE PEAK VOLTAGE
AND SUPPLY FLOW RATE
AS MINIMUM VALUE

DETERMINE SUPPLY FLOW RATE
BASED ON PEAK VOLTAGE ——S2600

PERFORM CONTROL
BASED ON DETERMINED PARAMETER ——S2700

END

FIG.18

VOLTAGE

Vmax

TRACK OF PEAK

Vmin

0        Tmin Tmax                                    Tc          TIME

**FIG.19**

STARTING-UP TIME (Tr)

Tmax    (S1' , T1')
(S2' , T1')
                (S1, T1)
                    (S2, T2)
                        (S4, T3)
Tmin

0                Sa                      Sb          SPEED
                                                     OF EJECTION PORT (S)

**FIG.20**

PEAK VOLTAGE (Vp)

Vmax

(S4, V3)

(S3, V2)

(S2, V1)

Vmin

0                              Sb          Sc      SPEED
                                                  OF EJECTION PORT (S)

# FIG.21

PEAK VOLTAGE,
SUPPLY FLOW RATE

SUPPLY FLOW RATE

PEAK VOLTAGE

0                                                 TIME

# FIG.22

| STARTING-UP TIME (ms) | MAXIMUM PRESSURE (MPa) | DEPTH OF RESECTION |
|---|---|---|
| 0.25 | 1.04 | - |
| 0.167 | 1.23 | 1 |
| 0.125 | 1.31 | 1.07 |
| 0.1 | 1.48 | 1.10 |

## FIG.23

## FIG.24

FIG.25

FIG.26

EJECTION TREATMENT (EMBODIMENT 5)

CALCULATE SPEED
OF NOZZLE — S3100

DETERMINE DRIVE VOLTAGE
AND DRIVE FREQUENCY
BASED ON SPEED OF NOZZLE — S3200

DETERMINE SUPPLY FLOW RATE
BASED ON DRIVE VOLTAGE
AND DRIVE FREQUENCY — S3300

PERFORM CONTROL
BASED ON DETERMINED PARAMETER — S3400

END

FIG.27

FIG.28

FIG.29

EJECTION TREATMENT (EMBODIMENT 6)

CALCULATE SPEED
OF NOZZLE — S4100

DETERMINE DRIVE VOLTAGE
BASED ON SPEED OF NOZZLE — S4210

S4220
IS DRIVE VOLTAGE
MAXIMUM VALUE?    NO

YES

S4230
DETERMINE DRIVE FREQUENCY
BASED ON SPEED OF NOZZLE

S4240
DETERMINE DRIVE FREQUENCY
AS MINIMUM VALUE

DETERMINE SUPPLY FLOW RATE
BASED ON DRIVE VOLTAGE
AND DRIVE FREQUENCY — S4300

PERFORM CONTROL
BASED ON DETERMINED PARAMETER — S4400

END

FIG.30

**EUROPEAN SEARCH REPORT**

Application Number

EP 14 19 2996

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2009/292165 A1 (SUGIYAMA YUTA [JP] ET AL) 26 November 2009 (2009-11-26) <br> * figures 1-4,6,10 * <br> * paragraphs [0050] - [0068], [0108] - [0138] * | 1,2,6-8, 13,14 | INV. <br> A61B19/00 <br> A61B17/3203 <br><br> ADD. <br> A61B17/00 |
| X <br> Y | US 2012/176431 A1 (KOJIMA HIDEKI [JP]) 12 July 2012 (2012-07-12) <br> * figures 5-10 * <br> * paragraphs [0007] - [0022], [0058] - [0088], [0103] - [0105], [0107] * | 15 <br><br> 1,2,6-8, 13,14 | |
| A | US 2013/116716 A1 (BAHLS THOMAS [DE] ET AL) 9 May 2013 (2013-05-09) <br> * paragraphs [0027], [0035] - [0041]; figures 1-6 * | 1-15 | |
| A | WO 2013/130895 A1 (AQUABEAM LLC [US]; ALJURI NIKOLAI [US]; MANTRI SURAG [US]; BAEZ LUIS [ ]) 6 September 2013 (2013-09-06) <br> * figures 24, 37-40 * <br> * paragraphs [0221] - [0022], [0417] - [0419], [0440] - [0443] * | 1-15 | TECHNICAL FIELDS SEARCHED (IPC) <br><br> A61B |
| A | US 2013/218184 A1 (SETO TAKESHI [JP] ET AL) 22 August 2013 (2013-08-22) <br> * figures 1-10 * <br> * paragraphs [0045] - [0110] * | 1-15 | |
| A | US 2011/037795 A1 (KOJIMA HIDEKI [JP] ET AL) 17 February 2011 (2011-02-17) <br> * the whole document * | 1-15 | |
| A | GB 2 495 248 A (DEZAC GROUP LTD [GB]) 3 April 2013 (2013-04-03) <br> * figures 3, 4 * <br> * page 4, line 14 - page 5, line 24 * <br> * page 11, line 2 - page 12, line 15 * | 1-3,6,7, 10,14,15 | |

-/--

The present search report has been drawn up for all claims

| Place of search | Date of completion of the search | Examiner |
|---|---|---|
| The Hague | 12 January 2015 | Friedrich, Franz |

EPO FORM 1503 03.82 (P04C01)

Europäisches
Patentamt

European
Patent Office

Office européen
des brevets

# EUROPEAN SEARCH REPORT

Application Number

EP 14 19 2996

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,P | US 2013/310862 A1 (SETO TAKESHI [JP] ET AL) 21 November 2013 (2013-11-21) * the whole document * ----- | 1-15 | |

TECHNICAL FIELDS
SEARCHED       (IPC)

The present search report has been drawn up for all claims

| Place of search | Date of completion of the search | Examiner |
|---|---|---|
| The Hague | 12 January 2015 | Friedrich, Franz |

EPO FORM 1503 03.82 (P04C01)

## ANNEX TO THE EUROPEAN SEARCH REPORT
## ON EUROPEAN PATENT APPLICATION NO.

EP 14 19 2996

This annex lists the patent family members relating to the patent documents cited in the above-mentioned European search report.
The members are as contained in the European Patent Office EDP file on
The European Patent Office is in no way liable for these particulars which are merely given for the purpose of information.

12-01-2015

| Patent document cited in search report | | Publication date | Patent family member(s) | | Publication date |
|---|---|---|---|---|---|
| US 2009292165 | A1 | 26-11-2009 | CN | 101594835 A | 02-12-2009 |
| | | | EP | 2108327 A1 | 14-10-2009 |
| | | | JP | 4960112 B2 | 27-06-2012 |
| | | | JP | 2008188109 A | 21-08-2008 |
| | | | US | 2009292165 A1 | 26-11-2009 |
| | | | WO | 2008093455 A1 | 07-08-2008 |
| US 2012176431 | A1 | 12-07-2012 | CN | 102579110 A | 18-07-2012 |
| | | | JP | 2012143374 A | 02-08-2012 |
| | | | US | 2012176431 A1 | 12-07-2012 |
| US 2013116716 | A1 | 09-05-2013 | DE | 102011086032 A1 | 16-05-2012 |
| | | | US | 2013116716 A1 | 09-05-2013 |
| WO 2013130895 | A1 | 06-09-2013 | CN | 104203078 A | 10-12-2014 |
| | | | EP | 2819599 A1 | 07-01-2015 |
| | | | WO | 2013130895 A1 | 06-09-2013 |
| US 2013218184 | A1 | 22-08-2013 | EP | 2022419 A2 | 11-02-2009 |
| | | | EP | 2821018 A1 | 07-01-2015 |
| | | | JP | 5115088 B2 | 09-01-2013 |
| | | | JP | 2009039384 A | 26-02-2009 |
| | | | US | 2009043480 A1 | 12-02-2009 |
| | | | US | 2013218184 A1 | 22-08-2013 |
| | | | US | 2014088628 A1 | 27-03-2014 |
| US 2011037795 | A1 | 17-02-2011 | EP | 2286745 A2 | 23-02-2011 |
| | | | JP | 4788809 B2 | 05-10-2011 |
| | | | JP | 2011036533 A | 24-02-2011 |
| | | | US | 2011037795 A1 | 17-02-2011 |
| GB 2495248 | A | 03-04-2013 | NONE | | |
| US 2013310862 | A1 | 21-11-2013 | CN | 103417265 A | 04-12-2013 |
| | | | US | 2013310862 A1 | 21-11-2013 |

EPO FORM P0459

For more details about this annex : see Official Journal of the European Patent Office, No. 12/82

**REFERENCES CITED IN THE DESCRIPTION**

**Patent documents cited in the description**

- JP 2012143374 A